# FASCICULE DE BREVET EUROPEEN

(11) **EP 0 599 732 B1**
(45) Date de publication et mention de la délivrance du brevet: **12.03.1997**
(21) Numéro de dépôt: 93402850.7
(22) Date de dépôt: 24.11.1993
(51) Int. Cl.: C07C 59/72, A61K 31/00, C07C 65/26, C07C 59/54

(54) **Dérivés de 1-phénylbicyclo[2.2.2]octane, leur procédé de préparation et les compositions pharmaceutiques qui les contiennent**
1-Phenybicyclo-2,2,2-oktanderivate, Verfahren zu ihrer Herstellung und sie enthaltende pharmazeutische Zusammensetzungen
Derivatives of 1-phenylbicyclo-2,2,2-octane, their preparation process and pharmaceutical compositions containing them

(30) Priorité: 24.11.1992 FR 9214058
(43) Date de publication de la demande: 01.06.1994
(73) Titulaire: ADIR ET COMPAGNIE, 92415 Courbevoie Cédex (FR)
(72) Inventeur: De Nanteuil, Guillaume, F-92150 Suresnes (FR); Vincent, Michel, F-92220 Bagneux (FR); Lila, Christine, F-78220 Viroflay (FR); Bonnet, Jacqueline, F-75013 Paris (FR); Fradin, Armel, F-92220 Neuilly Sur Seine (FR)

(56) Documents cités:
- FR-A- 2 251 316

## Description

La présente invention concerne de nouveaux dérivés de 1-phénylbicyclo[2.2.2]octane, leur procédé de préparation et les compositions pharmaceutiques qui les contiennent.

Ces composés, outre le fait qu'ils soient nouveaux, possèdent des propriétés pharmacologiques qui les rendent utilisables dans le traitement de l'arthrite.

Au cours de la réaction inflammatoire, d'importantes modifications se produisent dans la synthèse d'un groupe de protéines plasmatiques appelées les protéines de la phase aiguë. Certaines de ces protéines - dont le fibrinogène, la protéine C réactive, l'haptoglobine, le sérum amyloïde A, l'α₂-macroglobuline - sont augmentées au cours de la réaction de la phase aiguë, alors que d'autres comme l'albumine et la transférrine sont diminuées. L'altération de ces protéines, en particulier du fibrinogène, est à l'origine des modifications de la viscosité plasmatique et de la vitesse de sédimentation observées dans l'inflammation. En raison de leur corrélation avec les paramètres cliniques au cours de l'évolution et des rémissions thérapeutiques observées dans la polyarthrite rhumatoïde, certaines de ces protéines de la phase aiguë ont été utilisées comme critère d'évaluation de la maladie (Mallya RK & Coll., J. Rheumatol., 1982, 9, 224-8 ; Thompson PW & Coll., Arthritis Rheum 1987, 30, 618-23). Elles sont sous la dépendance des cytokines (Sipe JD, Interleukin 1 as a key factor in the acute-phase response. In : The acute-phase response to injury and infection. Edited by Gordon/Koj. Elsevier Science Publishers BV (Biomedical Division), 1985, p 23-35 ; Gauldie J & Coll., Cytokines and acute phase protein expression. In : Cytokines and Inflammation. Edited by ES Kimball. CRC Press, 1991, p 275-305) reconnues pour jouer un rôle important dans la pathologie arthritique.

En pharmacologie animale, les modifications des protéines de la phase aiguë ont été étudiées en particulier chez le rat au cours de la phase inflammatoire aiguë faisant suite à l'injection d'adjuvant complet (Lewis EJ & Coll., J. Pharmacol Meth 1989, 21, 183-94).

Plus spécifiquement, la présente invention concerne les composés de formule (I) : 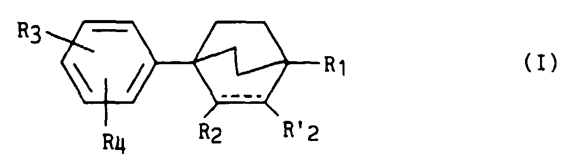 dans laquelle :
- R₁: représente un atome d'halogène, un groupement hydroxy, alkyle (C₁-C₆) linéaire ou ramifié (éventuellement substitué par un ou plusieurs atomes d'halogène), alkoxy (C₁-C₆) linéaire ou ramifié (éventuellement substitué par un ou plusieurs atomes d'halogène), cyano, amino (substitué ou non par un ou deux groupements alkyle (C₁-C₆) linéaire ou ramifié), mercapto, alkylthio (C₁-C₆) linéaire ou ramifié ou phénoxy (substitué ou non par un ou plusieurs atomes ou groupements, identiques ou différents, d'halogène, hydroxy, alkyle (C₁-C₆) linéaire ou ramifié, alkoxy (C₁-C₆) linéaire ou ramifié ou trihalogénoalkyle (C₁-C₆) linéaire ou ramifié),
- R₂ et R'₂: représentent deux atomes d'hydrogène dans le cas où le cycle bicyclooctane est insaturé,
ou bien
- R₂ ou R'₂,: dans le cas où le cycle bicyclooctane est saturé, identiques ou différents, représentent un atome d'hydrogène, d'halogène, un groupement hydroxy, alkyle (C₁-C₆) linéaire ou ramifié, alkoxy (C₁-C₆) linéaire ou ramifié, cyano, amino (substitué ou non par un ou deux groupements alkyle (C₁-C₆) linéaire ou ramifié) ou oxo,
- R₃: représente l'un quelconque des groupements suivants :
. -CO₂H (à la condition que dans ce cas, R₁ soit différent d'un atome d'halogène, d'un groupement alkyle (C₁-C₆) linéaire, ou amino),
. - CO - NH-CH₂ - CO₂H 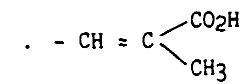
. - (CH₂)ₘ - CO₂H 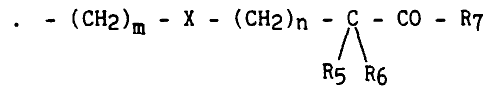 dans lesquels :
   m représente 1, 2 ou 3,
   X représente un atome d'oxygène, de soufre ou un groupement N-R (dans lequel R est un atome d'hydrogène ou un groupement alkyle (C₁-C₆) linéaire ou ramifié),
   R₅ ou R₆, identiques ou différents, représentent un atome d'hydrogène, un groupement alkyle (C₁-C₆) linéaire ou ramifié, trifluorométhyle
   ou 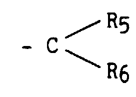 forme un radical cycloalkyle (C₃-C₆),
   n représente 0, 1 ou 2,
   R₇ représente un groupement hydroxy, alkoxy (C₁-C₆) linéaire ou ramifié, amino (substitué ou non par un ou deux groupements alkyle (C₁-C₆) linéaire ou ramifié), -O-CH₂-CO-NRR' (tels que R et R' représentent un groupement alkyle (C₁-C₆) linéaire ou ramifié, ou forment avec l'atome d'azote qui les portent un hétérocycle à 5 ou 6 chaînons),
. ou l'un quelconque des hétérocycles suivants : 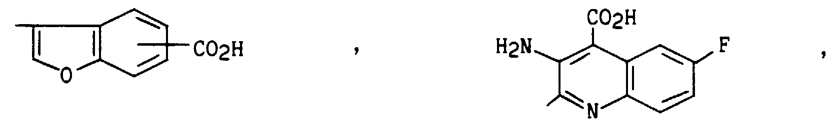 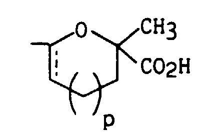 dans lequel p est égal à 0 ou 1, 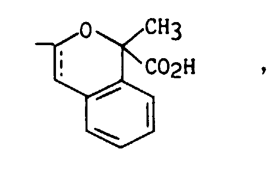
- R4: représente un atome d'hydrogène, d'halogène ou un groupement alkyle (C₁-C₆) linéaire ou ramifié, alkoxy (C₁-C₆) linéaire ou ramifié, trihalogénoalkyle (C₁-C₆),

les traits en pointillé sur les cycles indiquant la présence ou non d'une double liaison,
leurs énantiomères, diastéréoisomères et épimères ainsi que leurs sels d'addition à un acide ou à une base pharmaceutiquement acceptable.

Parmi les acides pharmaceutiquement acceptables, on peut citer à titre non limitatif les acides chlorhydrique, sulfurique, tartrique, maléique, fumarique, méthane sulfonique, camphorique, etc...

Parmi les bases pharmaceutiquement acceptables, on peut citer à titre non limitatif l'hydroxyde de sodium, l'hydroxyde de potassium, la tertbutylamine, la diéthylamine, l'éthylènediamine...

L'invention s'étend également au procédé de préparation des composés de formule (I) caractérisé en ce que l'on utilise comme produit de départ un composé de formule (II) : 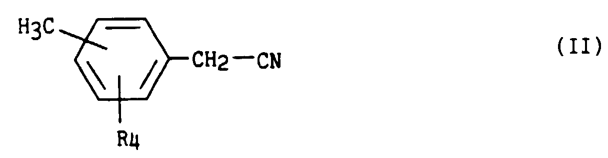 dans laquelle R₄ a la même signification que dans la formule (I),
que l'on fait réagir avec de l'acrylate de méthyle en présence d'une base pour donner le composé de formule (III) : 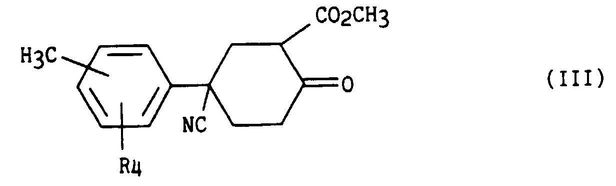 dans laquelle R₄ a la même signification que dans la formule (I),
que l'on soumet à l'action de l'acide paratoluènesulfonique dans l'éthylène glycol, pour conduire au composé de formule (IV) : 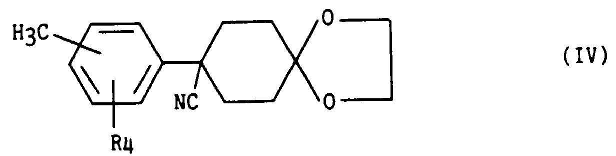 dans laquelle R₄ a la même signification que dans la formule (I),
qui subit l'action de l'iodure de méthyle magnésium, sous atmosphère inerte, pour conduire au composé de formule (V) : 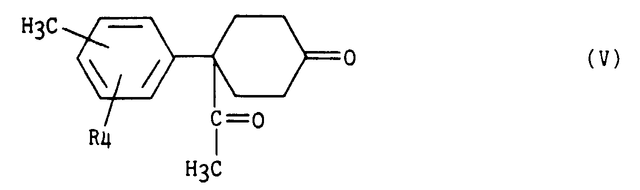 dans laquelle R₄ a la même signification que dans la formule (I),
que l'on fait réagir, en milieu anhydre, avec un alcool en milieu acide, pour conduire au composé de formule (VI) : 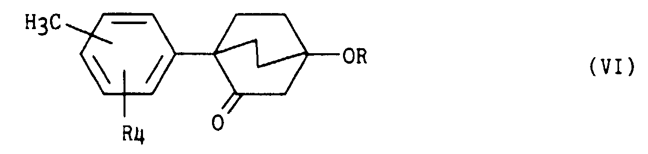 dans laquelle R₄ a la même signification que dans la formule (I) et R représente un groupement alkyle (C₁-C₆) linéaire ou ramifié,
composé de formule (VI) qui subit :
- soit une réduction totale en présence d'hydrate d'hydrazine et d'une base pour conduire au composé de formule (VIIa) : 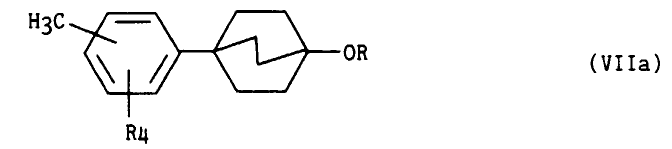 dans laquelle R₄ et R ont la même signification que précédemment,
- soit une réduction partielle en présence d'hydrure de lithium aluminium dans de l'éther suivie ou non d'une déshydratation pour conduire respectivement aux composés de formules (VIIb) et (VIIc) : 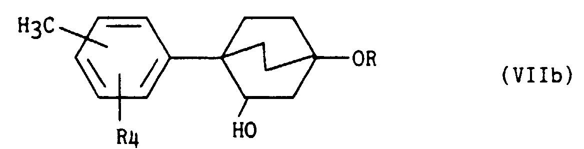 (composé de formule (VIIb) dont on transforme, si on le souhaite, le groupement hydroxy en atome d'halogène ou en groupement alkoxy, amino ou cyano, selon des techniques classiques de la chimie organique), 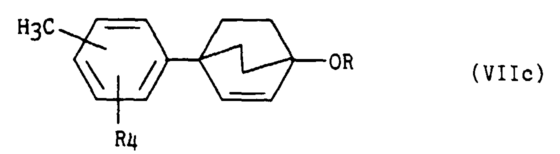 dans lesquelles R₄ et R ont la même signification que précédemment,
- soit l'action d'un ylure de phosphore suivie d'une réduction pour conduire au composé alkylé de formule (VIId) : 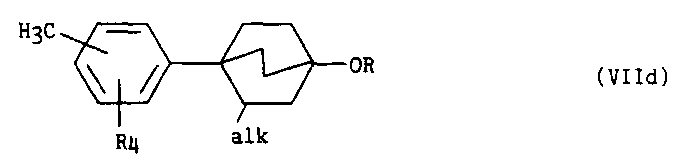 dans laquelle R₄ et R ont la même signification que précédemment et alk signifie alkyle (C₁-C₆) linéaire ou ramifié,
- soit, après transformation en énolate de lithium, une oxydation à l'aide de MoOPH (oxodiperoxymolybdenum-pyridine-hexaméthylphosphoramide) pour conduire au composé de formule (VIIe) : 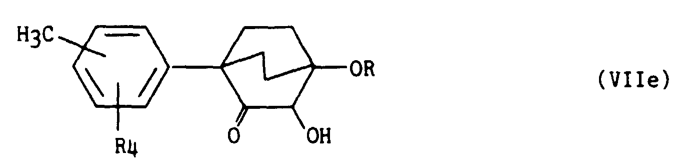 dans laquelle R₄ et R ont la même signification que précédemment (composé de formule (VIIe) dont on transforme, si on le souhaite, le groupement hydroxy en atome d'halogène ou en groupement alkoxy, amino ou cyano selon des techniques classiques de la chimie organique puis que l'on réduit éventuellement),
composés de formule (VIIa) à (VIIe) (dont on transforme, si on le souhaite, le groupement OR en groupement hydroxy ou en atome d'halogène puis amino, cyano, phénoxy, alkyle, mercapto, alkylthio, selon des techniques classiques de la chimie organique), qui constituent l'ensemble des composés de formule (VII) : 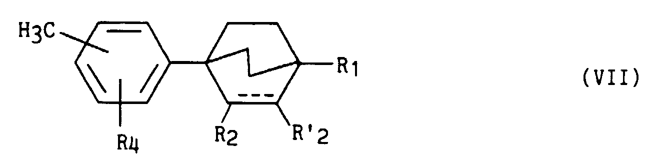 dans laquelle R₁, R₂, R'₂ et R₄ ont la même signification que dans la formule (I),
que l'on transforme en dérivé bromé correspondant de formule (VIIIa) par l'action du N-bromosuccinimide en milieu inerte : 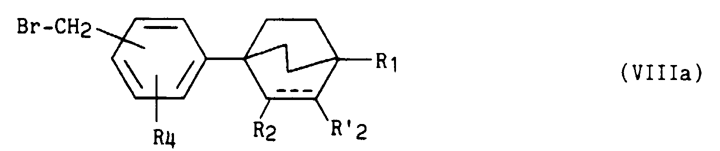 dans laquelle R₁, R₂, R'₂ et R₄ ont la même signification que dans la formule (I),
composé de formule (VIIIa), dont on transforme, éventuellement, le groupement -CH₂Br en groupement -CH₂CN correspondant puis en groupement -CH₂CO₂H, qui peut à nouveau subir des réactions classiques de la chimie organique conduisant :
- au composé de formule (I/a), cas particulier des composés de formule (I) : 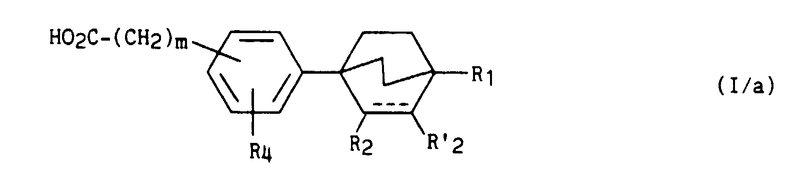 dans laquelle R₁, R₄, R₂, R'₂ et m ont la même signification que dans la formule (I),
- ou, au composé de formule (VIIIb) : 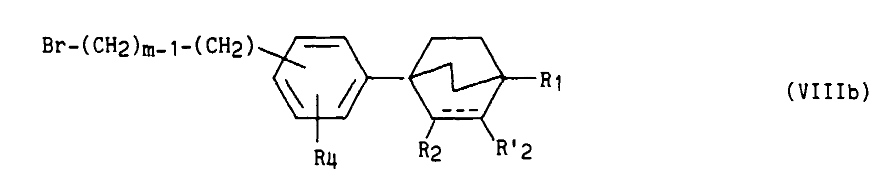 dans laquelle R₁, R₂, R'₂, R₄ et m ont la même signification que dans la formule (I),
composé de formule (VIIIa) ou (VIIIb) que l'on fait réagir :
a avec un composé de formule (IX) : 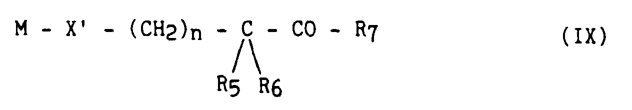 dans laquelle M représente un métal alcalin n, R₅, R₆ et R₇ sont tels que définis dans la formule (I) et X' représente un atome de soufre ou d'oxygène,
   pour conduire au composé de formule (I/b), cas particulier des composés de formule (I) : 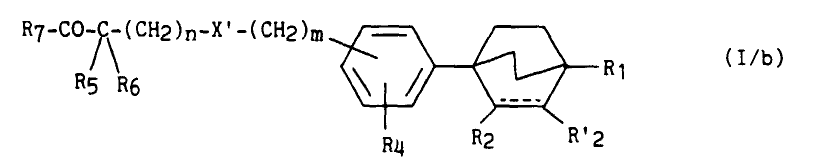 dans laquelle R₅, R₆, R₇, m, n, X', R₄, R₂, R'₂ et R₁ ont la même signification que précédemment,
   dont on transforme, si on le souhaite, R₇ lorsqu'il représente un groupement hydroxy en groupement amino ou ester correspondant selon les techniques classiques de transformation de fonction acide en fonction amide,
b avec de l'hexaméthylènetétramine en milieu chroroformique, puis en milieu acétique,
   pour conduire respectivement aux aldéhydes de formule (Xa) ou (Xb) : 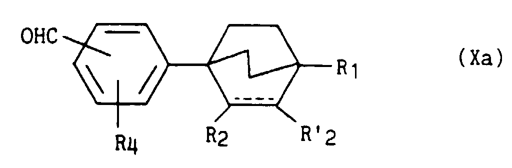 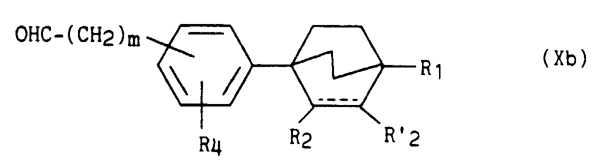
composé de formule (Xa) qui subit :
* soit l'action d'un oxydant comme le nitrate d'argent,
   pour conduire au composé de formule (I/c), cas particulier des composés de formule (I) : 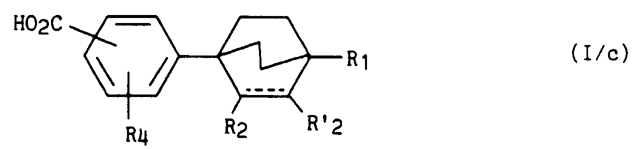 dans laquelle R₁, R₄, R₂ et R'₂ ont la même signification que précédemment,
   que l'on peut faire éventuellement réagir :
   - après transformation en chlorure d'acide, sur l'ylure de phosphore de formule (XI) : 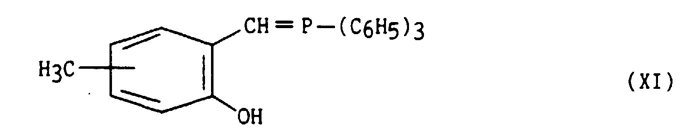 pour conduire au composé de formule (XII) : 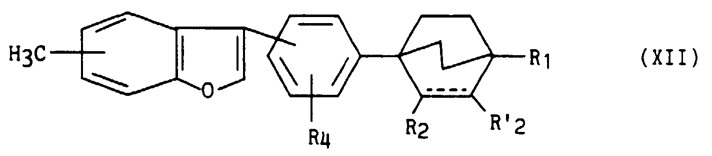 dans laquelle R₁, R₄, R₂ et R'₂ ont la même signification que précédemment,
      que l'on transforme en composé de formule (I/d), cas particulier des composés de formule (I), selon les mêmes réactions que celles décrites pour le passage du composé de formule (VII) en composé de formule (I/c) via les composés de formule (VIIIa) et (Xa) : 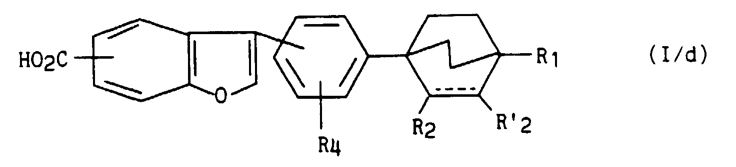 dans laquelle R₁, R₂, R'₂ et R₄ ont la même signification que précédemment,
   - après transformation en chlorure d'acide, sur le diazométhane pour conduire à la diazométhyl cétone correspondante que l'on transforme en bromométhylcétone puis en aminométhylcétone pour conduire, après réaction avec la 5-fluoroisatine, au composé de formule (I/e), cas particulier des composés de formule (I) : 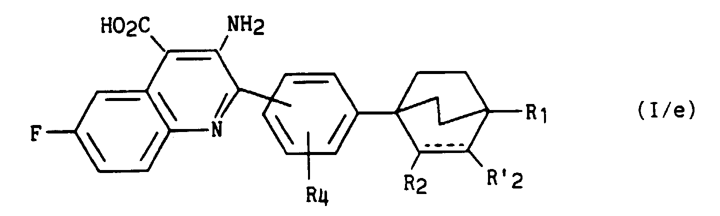 dans laquelle R₁, R₂, R'₂ et R₄ ont la même signification que dans la formule (I),
   - ou, sur l'ester méthylique de la glycine selon la technique classique de couplage peptidique décrite de W. KÖNIG et R. GEIGER (Chem. Ber., 103, 788, 2024, 1970),
      pour conduire au composé de formule (I/f), cas particulier des composés de formule (I) : 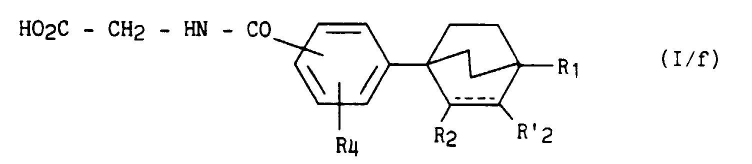 dans laquelle R₁, R₂, R'₂ et R₄ ont la même signification que dans la formule (I),
* soit l'action du magnésien C₆H₅CH₂MgBr suivie ou non d'une réaction d'oxydation puis de celle du pyruvate de méthyle en milieu acide, pour conduire après saponification au composé de formule (I/g), cas particulier des composés de formule (I) : 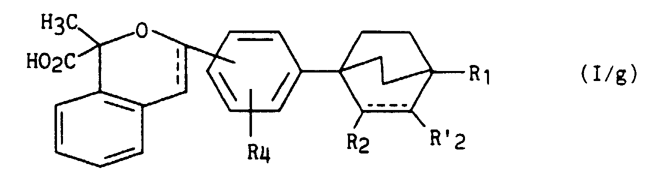 dans laquelle R₁, R₂, R'₂ et R₄ ont la même signification que dans la formule (I),
* soit l'action du magnésien CH₂=CH-MgBr suivie ou non d'une réaction d'oxydation puis de celle du pyruvate de méthyle en milieu acide pour conduire après saponification au composé de formule (I/h), cas particulier des composés de formule (I) : 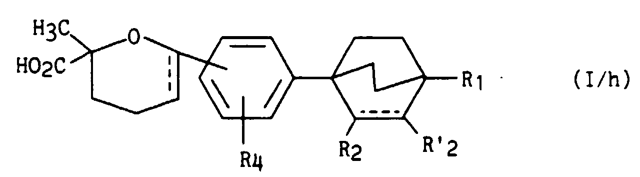 dans laquelle R₁, R₂, R'₂ et R₄ ont la même signification que dans la formule (I),
* soit l'action de : 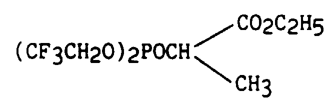 en présence d'une base, pour conduire après saponification au composé de formule (I/i) : 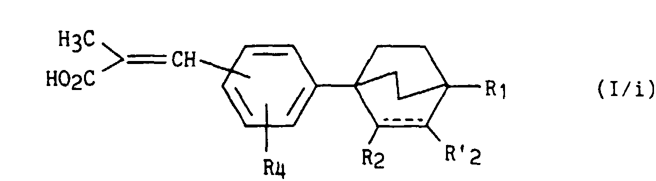 dans laquelle R₁, R₂, R'₂ et R₄ ont la même signification que dans la formule (I),
composés de formule (Xa) ou (Xb), qui peuvent subir l'action d'un amino ester de formule (XIII) : 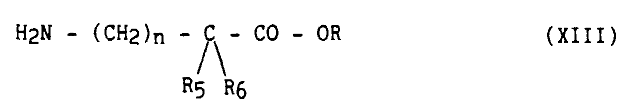 dans laquelle R représente un groupement alkyle, n, R₅ et R₆ ont la même signification que précédemment,
pour conduire au composé de formule (I/j), cas particulier des composés de formule (I) : 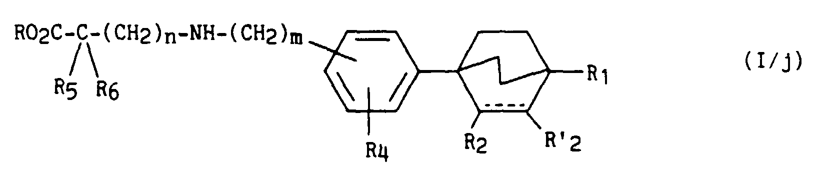 composé de formule (I/j) dont on transforme, si on le souhaite, la fonction acide ou fonction amide correspondante selon des techniques classiques de la chimie organique et la fonction amine secondaire en fonction amine tertiaire par alkylation,
composés de formule (I/a), (I/b), (I/c), (I/d), (I/e), (I/f), (I/g), (I/h), (I/i) ou (I/j) que l'on purifie, le cas échéant, selon une technique classique de purification dont on sépare, si on le souhaite, les isomères selon des techniques classiques de séparation, et que l'on transforme éventuellement en leurs sels d'addition à un acide ou à une base pharmaceutiquement acceptable.

Ces nouveaux dérivés de 1-phénylbicyclo[2.2.2]octane présentent des propriétés pharmacologiques très intéressantes. Ils diminuent les effets d'une injection d'adjuvant de Freund chez le rat tant au niveau des protéines plasmatiques de la phase aiguë (albumine) que de l'oedème aigu lui-même, tout en s'opposant à un stade plus tardif à l'apparition de la polyarthrite. Cet effet signale une activité antiinflammatoire des composés de l'invention.

L'invention s'étend aussi aux compositions pharmaceutiques renfermant comme principe actif au moins un composé de formule (I) ou ses isomères optiques avec un ou plusieurs excipients inertes, non toxiques et appropriés. Les compositions pharmaceutiques ainsi obtenues pourront être présentées sous diverses formes, les plus avantageuses étant les comprimés, les dragées, les gélules, suppositoires, suspensions buvables, les formes transdermiques (gel, patch), etc...

La posologie utile est adaptable selon la nature et la sévérité de l'affection, la voie d'administration ainsi que selon l'âge et le poids du patient. Cette posologie unitaire varie de 0,02 g à 2 g par jour en une ou plusieurs prises.

Les exemples suivants illustrent l'invention mais ne la limitent en aucune façon.

### EXEMPLE 1 : Acide 2-méthyl-2-{[3-(4-méthoxybicyclo[2.2.2]oct-1-yl)phényl] 1 : méthoxy} propionique, sel de sodium

### STADE A : 2-Méthoxycarbonyl-4-cyano-4-(3-méthylphényl)cyclohexan-1-one

Dans 64 ml d'une solution méthanolique contenant 198 mmoles de méthylate de sodium, sont ajoutées 190 mmoles de m-tolylacétonitrile puis goutte à goutte 380 mmoles d'acrylate de méthyle. L'ensemble est porté à reflux 3 heures. Après hydrolyse avec 200 ml d'acide chlorhydrique 1N, extraction à l'acétate d'éthyle puis lavage à l'eau et évaporation, le produit attendu est purifié par chromatographie sur colone de silice en utilisant comme éluant un mélange dichlorométhane/acétate d'éthyle (95/5).

### STADE B : 8-(3-Méthylphényl)-8-cyano-1,4-dioxy-spiro[4,5]décane

Un mélange contenant 73 mmoles du produit obtenu au stade précédent, 1,07 g d'acide paratoluènesulfonique, 11 ml d'eau et 110 ml d'éthylène glycol est porté à reflux pendant 4 heures. Après retour à température ambiante, hydrolyse par 150 ml d'une solution aqueuse saturée de chlorure d'ammonium, extraction par de l'éther, lavage, le produit attendu est obtenu sous forme d'huile après purification sur colonne de silice en utilisant comme éluant un mélange pentane/acétate d'éthyle (8/2).

### STACE C : 4-Acétyl-4-(3-méthylphényl)cyclohexan-1-one

145 ml d'une solution 3M d'iodure de méthyl magnésium dans de l'éther sont placés dans 100 ml de tétrahydrofurane anhydre sous atmosphère d'azote. L'ensemble est chauffé à 50°C puis sont ajoutées lentement 144 mmoles du produit obtenu au stade précédent en solution dans 100 ml de THF. L'ensemble est porté 24 heures à reflux. Après hydrolyse par 60 ml d'acide chlorhydrique concentré et 150 ml d'eau, le milieu est porté 12 heures à reflux. Après retour à température ambiante, extraction par de l'acétate d'éthyle, lavage et évaporation, le produit attendu est purifié par chromatographie sur colonne de silice en utilisant comme éluant un mélange dichlorométhane/acétate d'éthyle (97,5/2,5).

### STADE D : 2-Oxo-4-méthoxy-1-(3-méthylphényl)bicyclo[2.2.2]octane

Sur une solution à 0°C contenant 112 mmoles du composé obtenu au stade précédent dans 150 ml de méthanol anhydre, on fait passer un courant d'acide chlorhydrique pendant 3 heures. L'ensemble est maintenu sous agitation, à température ambiante pendant 12 heures. Après évaporation du solvant, l'ensemble est repris par de l'acétate d'éthyle, évaporé, séché et le produit attendu est obtenu après purification sur colonne de silice en utilisant comme éluant un mélange pentane/acétate d'éthyle (9/1).

### STADE E : 1-(3-Méthylphényl)-4-méthoxy bicyclo[2.2.2]octane

29 mmoles du produit obtenu au stade précédent en solution dans 35 ml d'hydrate d'hydrazine sont chauffées 5 heures à 160°C. Après addition de 8 g de potasse et de 50 ml d'éthylène glycol, l'ensemble est chauffé une heure à 160°C puis à 220°C jusqu'à la fin du dégagement d'azote. Après retour à température ambiante, l'ensemble est versé sur 100 ml d'eau et extrait par de l'éther. Après lavage, séchage et évaporation, on obtient le produit attendu.

### STADE F : 1-Méthoxy-4-(3-bromométhylphényl)bicyclo[2.2.2]octane

27 mmoles du composé obtenu au stade précédent sont placées dans 130 ml de tétrachlorure de carbone anhydre. Après addition d'un équivalent de N-bromosuccinimide puis de 0,1 équivalent de AIBN, l'ensemble est porté 2 heures au reflux. Après retour à température ambiante, puis refroidissement, l'ensemble est filtré et rincé. Le filtrat est évaporé et conduit au produit attendu.

### STADE G : 2-Méthyl-2-{[3-(4-méthoxy bicyclo[2.2.2]oct-1-yl)phényl]méthoxy} propionate d'éthyle

37,6 mmoles d'hydrure de sodium sont placées dans 33 mmoles de diméthylformamide anhydre sous atmosphère d'azote. On ajoute alors goutte à goutte 37,6 mmoles de 2-hydroxyisobutyrate d'éthyle dans 16 ml de DMF. L'ensemble est laissé une heure sous agitation puis refroidit à 0°C. 27 mmoles du composé obtenu au stade précédent dans 20 ml de DMF sont alors ajoutées et l'ensemble est abandonné 12 heures à température ambiante. On ajoute alors à 5°C, 15 ml d'une solution saturée de chlorure d'ammonium. Après évaporation du DMF, reprise par 200 ml d'acétate d'éthyle et 100 ml d'eau, la phase organique est extraite, séchée et évaporée. Le produit attendu est alors obtenu après purification sur colonne de silice en utilisant comme solvant un mélange pentane/acétate d'éthyle (90/10).

| Microanalyse élémentaire : | | |
|---|---|---|
| | C % | H % |
| calculé | 73,30 | 8,95 |
| trouvé | 73,42 | 8,79 |

### STADE H : Acide 2-méthyl-2-{[3-(4-méthoxy bicyclo[2.2.2]oct-1-yl) phényl]méthoxy} propionique, sel de sodium

A une solution contenant 8 mmoles du produit obtenu au stade précédent dans 60 ml d'éthanol, on ajoute 8 mmoles de soude en pastilles puis 4 ml d'eau. L'ensemble est porté 3 heures au reflux. Après évaporation, reprise du résidu par de l'eau, extraction par de l'éther, acidification par de l'acide chlorhydrique 4N, le produit attendu est obtenu après filtration et séchage et est transformé en sel de sodium correspondant.

| Microanalyse élémentaire : | | |
|---|---|---|
| | C % | H % |
| calculé | 67,78 | 7,68 |
| trouvé | 68,06 | 7,38 |

Les composés des exemples 2 et 3 ont été synthétisés selon le même procédé que celui décrit pour l'exemple 1 en utilisant les produits de départ correspondants.

### EXEMPLE 2 : Acide 2-méthyl-2-{[4-(4-méthoxybicyclo[2.2.2]oct-1-yl)phényl] méthoxy} propionique, sel de sodium

| Microanalyse élémentaire : | | |
|---|---|---|
| | C % | H % |
| calculé | 67,78 | 7,68 |
| trouvé | 67,65 | 7,37 |

### EXEMPLE 3 : Acide 2-méthyl-2-{[4-(4-éthoxybicyclo[2.2.2]oct-1-yl)phényl] méthoxy} propionique, sel de sodium

| Microanalyse élémentaire : | | |
|---|---|---|
| | C % | H % |
| calculé | 68,46 | 7,93 |
| trouvé | 68,47 | 7,50 |

### EXEMPLE 4 : 2-Méthyl-2-{[4-(4-méthoxybicyclo[2.2.2]oct-1-yl)phényl] méthoxy} propionate d'éthyle

Ce composé a été obtenu par estérification en milieu éthanolique du composé décrit dans l'exemple 2.

| Point de fusion : 52°C | | |
|---|---|---|
| Microanalyse élémentaire : | | |
| | C % | H % |
| calculé | 73,30 | 8,95 |
| trouvé | 73,40 | 8,31 |

### EXEMPLE 5 : Acide 2-méthyl-2-{[4-(4-chlorobicyclo[2.2.2]oct-1-yl)phényl] méthoxy} propionique, sel de sodium

Les stades A, B, C, D et E sont identiques aux stades A, B, C, D et E de l'exemple 2.

### STADE F : 1-(4-Méthylphényl)-4-hydroxybicyclo[2.2.2]octane

5 mmoles du produit obtenu au stade précédent sont portées au reflux d'une solution contenant 16 ml de dioxane et 16 ml d'acide chlorhydrique concentré pendant 24 heures. Après évaporation, le produit attendu est obtenu après lavage à l'eau, séchage.

### Point de fusion : 144°C

### STADE G : 1-(4-Méthylphényl)-4-chlorobicyclo[2.2.2]octane

4,6 mmoles du composé obtenu au stade précédent sont placées dans 10 ml de 1,2-dichloroéthane en présence de 9,2 mmoles de chlorure de thionyle. L'ensemble est porté 4 heures à reflux, évaporé et repris par du bicarbonate de soude et de l'acétate d'éthyle. Après extraction de la phase organique, lavage, séchage et évaporation, le produit attendu est obtenu sous forme de cristaux blancs.

### Point de fusion : 105°C

STADES H, I et J : Ces stades sont réalisés selon les procédés décrits dans les stades F, G et H de l'exemple 1 et conduisent au produit du titre.

| Microanalyse élémentaire : | | | |
|---|---|---|---|
| | C % | H % | Cl % |
| calculé | 63,60 | 6,74 | 9,88 |
| trouvé | 63,41 | 6,59 | 9,97 |

### EXEMPLE 6 : 2-{[4-(4-Chlorobicyclo[2.2.2]oct-1-yl)phényl]méthoxy} propionate d'éthyle

Ce composé a été obtenu par estérification en milieu éthanolique du composé de l'exemple 5.

| Microanalyse élémentaire : | | | |
|---|---|---|---|
| | C % | H % | Cl % |
| calculé | 69,12 | 8,01 | 9,72 |
| trouvé | 68,84 | 7,91 | 9,73 |

### EXEMPLE 7 : 2-Méthyl-2-{[4-(4-méthoxybicyclo[2.2.2]oct-1-yl)phényl] méthoxy} propionamide

### STADE A : Chlorure de l'acide 2-méthyl-2-{[4-(4-méthoxybicyclo[2.2.2]oct-1-yl)phényl]méthoxy} propionique

4,7 mmoles de l'acide décrit dans l'exemple 2 sont placées dans 10 ml de dichlorométhane anhydre en présence de quelques gouttes de diméthyl formamide. L'ensemble est refroidi à 0°C et 33 mmoles de chlorure d'oxalyle sont versées goutte à goutte sur ce mélange. Après 10 heures d'agitation à température ambiante, le produit attendu est obtenu après évaporation.

### STADE B : 2-Méthyl-2-{[4-(4-méthoxybicyclo[2.2.2]oct-1-yl)phényl]méthoxy} propionamide

4,7 mmoles du produit obtenu au stade précédent sont dissoutes dans 4 ml de dichlorométhane et placées dans 15 ml d'une solution ammoniacale à 28 % et l'ensemble est agité 3 heures à température ambiante. Le produit attendu est obtenu par filtration du précipité formé et séchage.

| Point de fusion : 186°C | | | |
|---|---|---|---|
| Microanalyse élémentaire : | | | |
| | C % | H % | N % |
| calculé | 72,47 | 8,82 | 4,23 |
| trouvé | 71,98 | 8,48 | 4,43 |

### EXEMPLE 8 : Acide [4-(4-hydroxybicyclo[2.2.2]oct-1-yl)phényl]acétique, sel de sodium

STADES A à F : Ces stades sont identiques aux stades A à F de l'exemple 2.

### STADE G : 1-Méthoxy-4-(4-cyanométhylphényl)bicyclo[2.2.2]octane

A 5,7 mmoles du produit obtenu au stade précédent dans 130 ml d'acétonitrile sont ajoutées 6,8 mmoles de cyanure de sodium en solution dans 18 ml d'eau. L'ensemble est porté 2 heures à reflux. Le produit attendu est obtenu après évaporation de l'acétonitrile, repris par un mélange eau-éther, extraction séchage et évaporation.

### STADE H : Acide [4-(4-hydroxybicyclo[2.2.2]oct-1-yl)phényl]acétique, sel de sodium

A 5 mmoles du composé obtenu au stade précédent dans 18 ml de dioxane sont ajoutés, goutte à goutte, 18 ml d'acide chlorhydrique concentré. L'ensemble est porté 3 heures à reflux. Le précipité formé est filtré, lavé à l'eau et séché et est transformé en sel de sodium correspondant.

| Microanalyse élémentaire : | | |
|---|---|---|
| | C % | H % |
| calculé | 68,07 | 6,78 |
| trouvé | 68,33 | 6,63 |

### EXEMPLE 9 : Acide 4-(4-méthoxybicyclo[2.2.2]oct-1-yl)benzoïque, sel de sodium

STADES A à F : ces stades sont identiques aux stades A à F de l'exemple 2.

### STADE G : Bromure de N-[4-(4-méthoxybicyclo[2.2.2]oct-1-yl)benzyl] hexaméthylènetétramonium

68 mmoles d'hexaméthylènetétramine sont dissoutes à reflux dans 100 ml de chloroforme. 68 mmoles du composé obtenu au stade précédent en solution dans 30 ml de chloroforme sont alors ajoutées. L'ensemble est maintenu 4 heures à reflux et le produit attendu est obtenu sous forme de précipité qui est filtré et séché.

### STADE H : 4-(4-Méthoxybicyclo[2.2.2]oct-1-yl)benzaldéhyde

15 mmoles du composé obtenu au stade précédent sont placées dans 44 ml d'une solution acide acétique/eau (1/1). L'ensemble est chauffé 3 heures à 100°C. 10 ml d'acide chlorhydrique 6N sont alors ajoutés et l'agitation est maintenu 30 minutes. Après retour à température ambiante, le produit attendu est obtenu après extraction par de l'éther et purifié par chromatographie sur colonne de silice en utilisant comme éluant un mélange pentane/acétate d'éthyle (9/1).

| Microanalyse élémentaire : | | |
|---|---|---|
| | C % | H % |
| calculé | 78,65 | 8,25 |
| trouvé | 78,87 | 8,40 |

### STADE I : Acide 4-(4-méthoxybicyclo[2.2.2]oct-1-yl)benzoïque, sel de sodium

A l'abri de la lumière, 46 mmoles de soude dans 5 ml d'eau sont refroidies à 0°C, puis on ajoute une solution contenant 21 mmoles de nitrate d'argent dans 5 ml d'eau. 8,2 mmoles du produit obtenu au stade précédent sont alors ajoutées lentement. L'ensemble est maintenu 10 heures sous agitation. Après filtration, rinçage à l'eau chaude, refroidissement du filtrat, le produit attendu sous forme d'acide libre est obtenu par précipitation à l'aide d'acide chlorhydrique 4N et filtration et est transformé en sel de sodium correspondant.

| Microanalyse élémentaire : | | |
|---|---|---|
| | C % | H % |
| calculé | 68,07 | 6,78 |
| trouvé | 67,67 | 6,39 |

### EXEMPLE 10 : Acide 2-méthyl-2-{[4-(2-hydroxy-4-méthoxybicyclo[2.2.2]oct-1-yl)phényl]méthoxy} propionique, sel de sodium

STADES A à D : ces stades sont identiques aux stades A à D de l'exemple 2.

STADE E : 1-Méthoxy-3-hydroxy-4-(4-méthylphényl)bicyclo[2.2.2]octane 8,2 mmoles du composé obtenu au stade précédent en solution dans 10 ml de tétrahydrofurane anhydre sont ajoutées goutte à goutte à une suspension à 5°C contenant 10,6 mmoles de LiAlH₄ dans 20 ml de THF anhydre. Après une heure à 5°C puis 2 heures à température ambiante, l'ensemble est à nouveau refroidi à 5°C puis hydrolysé. Après filtration, évaporation du filtrat, le résidu est repris par de l'éther et de l'eau et extrait à l'éther et conduit au produit attendu.

| Microanalyse élémentaire : | | |
|---|---|---|
| | C % | H % |
| calculé | 78,01 | 9,00 |
| trouvé | 78,05 | 8,85 |

### STADE F : 1-Méthoxy-2-acétyloxy-4-(4-méthylphényl)bicyclo[2.2.2]octane

7,5 mmoles du composé obtenu au stade précédent en solution dans 20 ml de chloroforme sont acétylées en présence de 1,1 équivalent de chlorure d'acétyle et de 1,1 équivalent de pyridine. Le produit attendu est alors obtenu après évaporation et extraction à l'éther.

| Microanalyse élémentaire : | | |
|---|---|---|
| | C % | H % |
| calculé | 74,97 | 8,39 |
| trouvé | 75,19 | 8,18 |

STADES G à I : ces stades sont identiques aux stades F à H de l'exemple 2.

| Microanalyse élémentaire : | | |
|---|---|---|
| | C % | H % |
| calculé | 64,85 | 7,35 |
| trouvé | 64,55 | 6,94 |

Les exemples 11 et 12 ont été synthétisés selon le même procédé que celui décrit pour l'exemple 1 en utilisant les produits de départ correspondants.

### EXEMPLE 11 : {2-Méthyl-2-[[4-(4-méthoxybicyclo[2.2.2]oct-1-yl)phényl] méthoxy]propionyloxy}-N,N-diéthylacétamide

| Point de fusion : 94°C | | | |
|---|---|---|---|
| Microanalyse élémentaire : | | | |
| | C % | H % | N % |
| calculé | 70,08 | 8,82 | 3,14 |
| trouvé | 70,22 | 8,78 | 3,51 |

### EXEMPLE 12 : {2-Méthyl-2-[[3-(4-méthoxybicyclo[2.2.2]oct-1-yl)phényl] méthoxy]propionyloxy}-N,N-diéthylacétamide

| Point de fusion : 84°C | | | |
|---|---|---|---|
| Microanalyse élémentaire : | | | |
| | C % | H % | N % |
| calculé | 70,08 | 8,82 | 3,14 |
| trouvé | 69,53 | 8,71 | 3,31 |

### EXEMPLE 13 : Acide 2-méthyl-2-{[4-(-4-méthoxybicyclo[2.2.2]oct-1-yl) phényl]méthylamino} propionique, sel de sodium

Les stades A à H sont identiques aux stades A à H de l'exemple 9.

### STADE I : 2-Méthyl-2-{[4-(4-méthoxybicyclo[2.2.2]oct-1-yl)phényl] méthylamino} propionate d'éthyle

A 7 mmoles de 2-amino-2-méthyl propionate d'éthyle en solution dans 30 ml d'éthanol sont ajoutées 22 g de tamis moléculaire (3Å), 8,6 mmoles de triéthylamine puis 7,8 mmoles du composé obtenu au stade précédent en solution dans 30 ml d'éthanol. L'ensemble est maintenu sous agitation 90 minutes, puis on ajoute 1,3 ml d'acide acétique et 1 équivalent de cyanoborohydrure de sodium et l'agitation est maintenue 17 heures. Le tamis moléculaire est filtré, rincé à l'éther et le filtrat évaporé. Le résidu est repris par de l'eau et extrait par de l'acétate d'éthyle. Le produit attendu est obtenu après purification par chromatographie sur gel de silice en utilisant comme éluant un mélange dichlorométhane/acétate d'éthyle (70/30).

| Microanalyse élémentaire : | | |
|---|---|---|
| | C % | H % |
| calculé | 69,55 | 7,30 |
| trouvé | 69,82 | 7,25 |

### STADE J : Acide 2-méthyl-2-{[4-(-4-méthoxybicyclo[2.2.2]oct-1-yl)phényl] méthylamino} propionique, sel de sodium

L'ester obtenu au stade précédent est saponifié en milieu éthanolique puis transformé en sel de sodium correspondant.

| Microanalyse élémentaire : | | | |
|---|---|---|---|
| | C % | H % | N % |
| calculé | 67,97 | 7,99 | 3,96 |
| trouvé | 67,93 | 7,48 | 3,97 |

### EXEMPLE 14 : Acide 2-[4-(4-méthoxybicyclo[2.2.2]oct-1-yl)phényl] benzofurane-7-carboxylique, sel de sodium

### STADE A : 6-Méthyl-2-hydroxyméthylphénol

4,9 mmoles d'hydrure de lithium aluminium sont placées sous atmosphère d'azote dans 50 ml d'éther. 3,3 mmoles d'acide 2-hydroxy-3-méthyl benzoïque en solution dans 40 ml d'éther sont ajoutées goutte à goutte en maintenant un reflux pendant 4 heures. L'ensemble est alors refroidi dans de la glace et 12,5 ml d'isopropanol puis 7,5 ml d'une solution saturée de chlorure de sodium sont ajoutés. Après 10 heures à température ambiante, l'ensemble est filtré, rincé à l'acide chlorhydrique 1N et extrait à l'éther. Le produit est alors obtenu après purification sur colonne de silice en utilisant comme éluant un mélange pentane/acétate d'éthyle (7/3).

| Microanalyse élémentaire : | | |
|---|---|---|
| | C % | H % |
| calculé | 69,55 | 7,30 |
| trouvé | 69,82 | 7,25 |

### STADE B : Bromure de 3-méthyl-2-hydroxy benzyl phosphonium

10 mmoles du produit obtenu au stade précédent et 10 mmoles de bromure de triphénylphosphonium dans 10 ml d'acétonitrile sont portées à 100°C pendant 2 heures. Le produit attendu précipite, est filtré, rincé à l'acétate d'éthyle et séché.

| Microanalyse élémentaire : | | | |
|---|---|---|---|
| | C % | H % | Br % |
| calculé | 67,40 | 5,22 | 17,24 |
| trouvé | 67,58 | 5,14 | 17,70 |

### STADE C : 7-Méthyl-2-[4-(4-méthoxybicyclo[2.2.2]oct-1-yl)phényl] benzofurane

A 37 mmoles de chlorure de l'acide décrit dans l'exemple 9 (obtenu par réaction du pentachlorure de phosphore sur le composé de l'exemple 9) en solution dans 120 ml de toluène, sont ajoutées 31,4 mmoles du composé obtenu au stade précédent et 13,2 ml de triéthylamine. L'ensemble est porté à reflux pendant 8 heures. Après filtration, le filtrat est évaporé, repris à l'éthanol et conduit au produit attendu par évaporation.

Les stades D à G sont réalisés en utilisant les mêmes procédés que ceux décrits dans les stades F à I de l'exemple 9.

| Microanalyse élémentaire : | | |
|---|---|---|
| | C % | H % |
| calculé | 72,35 | 5,82 |
| trouvé | 71,62 | 5,55 |

### EXEMPLE 15 : Acide 1-{[4-(4-méthoxybicyclo[2.2.2]oct-1-yl)phényl] méthylamino} cyclopropane carboxylique

Ce composé a été obtenu selon le même procédé que celui décrit dans l'exemple 13.

| Microanalyse élémentaire : | | | |
|---|---|---|---|
| | C % | H % | N % |
| calculé | 68,36 | 7,46 | 3,99 |
| trouvé | 68,20 | 6,96 | 4,20 |

### EXEMPLE 16 : Acide 2-méthyl-2-{[4-(4-méthoxybicyclo[2.2.2]oct-1-yl) phényl]méthoxy} butanoïque, sel de sodium

Ce composé a été obtenu selon le procédé décrit dans l'exemple 1.

| Microanalyse élémentaire : | | |
|---|---|---|
| | C % | H % |
| calculé | 68,46 | 7,93 |
| trouvé | 68,95 | 7,64 |

### EXEMPLE 17 : Acide 2-méthyl-2-{[4-(4-hydroxybicyclo[2.2.2]oct-1-yl) phényl]méthoxy}propionique, sel de sodium

Les stades A à F sont identiques aux stades A à F de l'exemple 5.

### STADE G : 1-(4-Bromométhylphényl)-4-hydroxybicyclo[2.2.2]octane

Le produit attendu est obtenu selon le procédé décrit au stade F de l'exemple 1 à partir du 1-(4-méthylphényl)-4-hydroxybicyclo[2.2.2]octane obtenu au stade précédent.

STADES H et I : ces stades sont réalisés selon les procédés décrits dans les stades G et H de l'exemple 1 et conduisent au produit du titre.

| Microanalyse élémentaire : | | |
|---|---|---|
| | C % | H % |
| calculé | 67,04 | 7,40 |
| trouvé | 67,35 | 7,09 |

### EXEMPLE 18 : Acide 2-méthyl-2-{[4-(4-isopropyloxybicyclo[2.2.2]oct-1-yl)phényl]méthoxy}propionique, sel de sodium

Ce composé a été obtenu selon le procédé décrit dans l'exemple 1 à partir des produits de départ correspondants.

| Microanalyse élémentaire : | | |
|---|---|---|
| | C % | H % |
| calculé | 69,09 | 8,17 |
| trouvé | 69,03 | 7,96 |

### EXEMPLE 19 : Acide 2-méthyl-2-{[4-(4-méthoxybicyclo[2.2.2]oct-1-yl) phényl]méthoxy}propionique

Ce composé est obtenu par déplacement en milieu chlorhydrique du sel de sodium correspondant décrit dans l'exemple 2.

### Point de fusion : 169-170°C

| Microanalyse élémentaire : | | |
|---|---|---|
| | C % | H % |
| calculé | 72,26 | 8,49 |
| trouvé | 71,70 | 8,27 |

### EXEMPLE 20 : Acide 2-éthyl-2-{[4-(4-méthoxybicyclo[2.2.2]oct-1-yl) phényl]méthoxy}butyrique, sel de sodium

Ce composé a été obtenu selon le procédé décrit dans l'exemple 1 à partir des produits de départ correspondants.

| Microanalyse élémentaire : | | |
|---|---|---|
| | C % | H % |
| calculé | 69,09 | 8,17 |
| trouvé | 69,46 | 8,12 |

### EXEMPLE 21 : Acide 2-{[4-(4-méthoxybicyclo[2.2.2]oct-1-yl)phényl) méthoxy}propionique, sel de sodium

Ce composé a été obtenu selon le procédé décrit dans l'exemple 1 en utilisant les produits de départ correspondants.

| Microanalyse élémentaire : | | |
|---|---|---|
| | C % | H % |
| calculé | 67,04 | 7,40 |
| trouvé | 66,84 | 7,38 |

### EXEMPLE 22 : Acide 1-{[4-(4-méthoxybicyclo[2.2.2]oct-1-yl)phényl] méthoxy}cyclopentanecarboxylique, sel de sodium

Ce composé a été obtenu selon le procédé décrit dans l'exemple 1 en utilisant les produits de départ correspondants.

| Microanalyse élémentaire : | | |
|---|---|---|
| | C % | H % |
| calculé | 69,45 | 7,68 |
| trouvé | 69,78 | 7,86 |

### EXEMPLE 23 : N-[4-(4-Méthoxybicyclo[2.2.2]oct-1-yl)benzoyl]glycine, sel de sodium

Le produit attendu est obtenu par couplage peptidique DCC/HOBT selon la technique décrite par W. KONIG et R. GEIGER (Chem. Ber., 103, 788, 2024, 1970), à partir du composé décrit dans l'exemple 9 sous forme d'acide libre et de l'ester méthylique de la glycine, suivie d'une saponification en milieu méthanol/soude.

| Microanalyse élémentaire : | | | |
|---|---|---|---|
| | C % | H % | N % |
| calculé | 63,71 | 6,53 | 4,13 |
| trouvé | 63,92 | 6,56 | 4,46 |

### EXEMPLE 24 : Acide 2-méthyl-2-{2-[4-(4-méthoxybicyclo[2.2.2]oct-1-yl) phényl]éthoxy}propionique, sel de sodium

STADES A à G : ces stades sont identiques aux stades A à G de l'exemple 8.

### STADE H : Acide [4-(4-méthoxybicyclo[2.2.2]oct-1-yl)phényl]acétique

50 mmoles du composé obtenu au stade précédent, 130 ml de potasse à 20 % et 130 ml d'éthylène glycol sont portés à reflux pendant 5 heures. Après filtration et refroidissement, le filtrat est acidifié et le produit attendu est obtenu après filtration du précipité, lavage à l'eau et séchage.

| Microanalyse élémentaire : | | |
|---|---|---|
| | C % | H % |
| calculé | 74,42 | 8,08 |
| trouvé | 74,33 | 8,22 |

### STADE I : 2-[4-(4-Méthoxybicyclo[2.2.2]oct-1-yl)phényl]éthanol

A 100 ml de tétrahydrofurane anhydre, sous atmosphère d'argon, sont ajoutées 55 mmoles d'hydrure de lithium aluminium puis à température ambiante, goutte à goutte, une solution contenant 37 mmoles du composé obtenu au stade précédent dans 300 ml de THF anhydre. L'ensemble est porté 3 heures à reflux. Après refroidissement, hydrolyse avec 9,4 ml d'isopropanol et 5,6 ml d'une solution saturée de chlorure de sodium, l'ensemble est agité une nuit. Le précipité est essoré, lavé au THF, repris par de l'acétate d'éthyle, lavé au bicarbonate de soude puis à l'eau et enfin après évaporation, filtré et séché et conduit au produit attendu.

### STADE J : Acide 2-méthyl-2-{-2-[4-(4-méthoxybicyclo[2.2.2]oct-1-yl)phényl] éthoxy}propionique, sel de sodium

A une solution contenant 29 mmoles de composé obtenu au stade précédent dans 43 ml d'acétone anhydre sont ajoutées 144 mmoles de soude en poudre. L'ensemble est porté à reflux et on ajoute au reflux 3 ml de chloroforme dans 8,7 ml d'acétone anhydre. Le reflux est maintenu 4 heures. Après refroidissement et évaporation, le résidu est repris par un mélange eau/acétate d'éthyle. Après acidification, les cristaux formés sont filtrés, rincés et séchés et conduisent au produit attendu après transformation en sel de sodium correspondant.

| Microanalyse élémentaire : | | |
|---|---|---|
| | C % | H % |
| calculé | 68,46 | 7,93 |
| trouvé | 68,60 | 7,68 |

### EXEMPLE 25 : Acide 2-méthyl-2-{[4-(4-méthoxybicyclo[2.2.2]oct-1-yl) phényl]méthylthio}propionique, sel de sodium

STADES A à F : ces stades sont identiques aux stades A à F de l'exemple 2.

### STADE G : {[4-(4-Méthoxybicyclo[2.2.2]oct-1-yl)phényl]méthylthio} méthylcétone

Dans un tricol protégé de la lumière, sont placés 19,8 g de carbonate de césium dans 100 ml de diméthylformamide anhydre puis 8,06 ml d'acide thioacétique dans 50 ml de diméthylformamide anhydre. L'ensemble est agité, à température ambiante, jusqu'à dissolution complète, puis refroidi dans un bain glace-eau. 86 mmoles du composé obtenu au stade précédent dans 60 ml de DMF anhydre sont alors ajoutées et l'ensemble est maintenu une nuit sous agitation à température ambiante. Après évaporation, reprise par de l'acétate d'éthyle, lavage par de l'acide chlorhydrique 0,1N puis par une solution saturée de chlorure de sodium et évaporation, le produit attendu est obtenu par purification du résidu par chromatographie sur colonne de silice en utilisant comme éluant un mélange acétate d'éthyle/pentane (1/9).

### STADE H : 2-Méthyl-2-{[4-(4-méthoxybicyclo[2.2.2]oct-1-yl)phényl] méthylthio}propionate d'éthyle

20 mmoles du composé obtenu au stade précédent sont placées, sous atmosphère d'azote, dans 50 ml d'éthanol anhydre. 46 mg de sodium sont alors ajoutés par portions. Après 15 minutes de contact, 20 mmoles de 2-bromoisobutyrate d'éthyle en solution dans 20 ml d'éthanol anhydre sont ajoutées lentement. L'ensemble est porté une heure à reflux. Après 2 jours à température ambiante, 1,1 équivalent d'acide chlorhydrique 1N sont ajoutés. Le précipité formé est filtré, rincé au méthanol et conduit au produit attendu.

### STADE I : Acide 2-méthyl-2-{[4-(4-méthoxybicyclo[2.2.2]oct-1-yl)phényl] méthylthio}propionique, sel de sodium

Le produit attendu est obtenu selon le procédé décrit au stade H de l'exemple 1.

| Microanalyse élémentaire : | | | |
|---|---|---|---|
| | C % | H % | S % |
| calculé | 64,84 | 7,35 | 8,65 |
| trouvé | 65,10 | 7,40 | 8,08 |

### EXEMPLE 26 : Acide 3-amino-6-fluoro-2-[4-(4-méthoxybicyclo[2.2.2]oct-1-yl)phényl]quinoléine-4-carboxylique, sel de sodium

### STADE A : [4-(4-Méthoxybicyclo[2.2.2]oct-1-yl)phényl](chlorométhyl)cétone

42 mmoles du chlorure d'acide du composé décrit dans l'exemple 8 dans 100 ml de THF anhydre sont refroidies dans un bain de glace. 230 ml d'une solution 0,32N de diazométhane dans de l'éther sont alors ajoutés goutte à goutte. L'ensemble est agité 30 minutes à 0°C puis 3 h 30 à température ambiante. L'excès de diazométhane est alors chassé sous courant d'azote. Après refroidissement au bain de glace, on fait passer un courant d'acide chlorhydrique gaz pendant 10 minutes puis l'ensemble est à nouveau laissé 10 minutes à 0°C. Après évaporation, on obtient le produit attendu.

### STADE B : [4-(4-Méthoxybicyclo[2.2.2]oct-1-yl)phényl] (diformylamidométhyl)cétone

A 20 mmoles du composé obtenu au stade précédent dans 20 ml d'acétonitrile sont ajoutées 23 mmoles de sodium diformylamide. L'ensemble est porté 4 heures à reflux. Le produit attendu est alors obtenu après filtration et évaporation du filtrat.

### STADE C : [4-(4-Méthoxybicyclo[2.2.2]oct-1-yl)phényl](aminométhyl)cétone, chlorhydrate

15 mmoles du composé obtenu au stade précédent dans 37 ml d'une solution chlorhydrique d'éthanol à 5 % sont agitées 6 jours à température ambiante. Après évaporation, on obtient le produit attendu.

### STADE D : Acide 3-amino-6-fluoro-2-[4-(4-méthoxybicyclo[2.2.2]oct-1-yl) phényl]quinoléine-4-carboxylique, sel de sodium

17,4 mmoles de 5-fluoroisatine sont placées dans 25 ml d'eau. 95 mmoles de soude en pastilles dans 15 ml d'eau sont alors ajoutées. L'ensemble est porté à 90°C et 24 mmoles du composé obtenu au stade précédent dans 80 ml d'un mélange éthanol/eau (1/1) sont alors ajoutées, lentement, à cette température qui est ensuite maintenue 2 heures. Après refroidissement, évaporation de l'éthanol et filtration, le filtrat est acidifié à pH 2,5 avec de l'acide acétique. Le précipité est filtré, lavé à l'eau et après transformation en sel de sodium, conduit au produit attendu.
Spectre de masse : FAB⁺ / [MH]⁺ m/z = 443

### EXEMPLE 27 : Acide (Z) 2-méthyl-[4-(4-méthoxybicyclo[2.2.2]oct-1-yl) phényl]prop-2-ènoïque, sel de sodium

STADES A à H : ces stades sont identiques aux stades A à H de l'exemple 9.

### STADE I : 2-Méthyl-[4-(4-méthoxybicyclo[2.2.2]oct-1-yl)phényl]prop-2-ènoate d'éthyle

10 mmoles de (CF₃CH₂O)₂PO-CHCH₃CO₂CH₂CH₃ et 10 mmoles d'éther couronne 18-crown-6 sont placées dans 150 ml de THF anhydre à -78°C. On ajoute alors 20 ml d'une solution 0,5M de potassium hexaméthyldisilazane dans du toluène puis 10 mmoles du composé obtenu au stade précédent dans 50 ml de THF anhydre. L'ensemble est maintenu 2 heures à -78°C, évaporé, repris par un mélange eau/acétate d'éthyle. La phase organique est alors lavée, séchée et évaporée et le produit attendu est obtenu après purification du résidu sur colonne de silice en utilisant comme éluant un mélange pentane/acétate d'éthyle 95/5.

### STADE J : Acide (Z) 2-méthyl-[4-(4-méthoxybicyclo[2.2.2]oct-1-yl) phényl]prop-2-ènoïque, sel de sodium

7 mmoles du composé obtenu au stade précédent sont placées dans 10 ml de méthanol en présence de 7,7 mmoles de soude en pastilles et portées à reflux 3 heures. Après filtration, évaporation, reprise par de l'eau, extraction à l'éther et évaporation, le produit attendu est obtenu après transformation de l'acide en sel de sodium correspondant.

| Microanalyse élémentaire : | | |
|---|---|---|
| | C % | H % |
| calculé | 70,79 | 7,19 |
| trouvé | 71,24 | 7,15 |

### Etude pharmacologique des dérivés de l'invention

### EXEMPLE 28 : Activité in vivo sur les protéines de la phase aiguë

L'activité biologique des composés de l'invention a été déterminée en particulier sur l'albumine plasmatique du rat 6 jours après injection sous-cutanée d'adjuvant complet de Freund. Protéine négative de la phase aiguë de l'inflammation, l'albumine, fortement diminuée par l'état inflammatoire qui fait suite à l'adjuvant, est rétablie en tout ou partie par les composés administrés à la dose orale quotidienne de 100 mg/kg.

### Protocole expérimental

L'arthrite à l'adjuvant chez le rat, pour la première fois décrite par Pearson (Pearson CM., Proc. Soc. Exp. Biol. Med., 1956, 91, 95-101) a été provoquée par injection de 0,1 ml d'adjuvant complet de Freund (4 mg de Mycobacterium butyricum en suspension dans 1 ml d'huile de paraffine/eau/tween 80) dans la région sous-plantaire de la patte postérieure du rat femelle Lewis (âgé de 62 jours). Les produits ont été administrés quotidiennement sous forme de solution aqueuse ou de suspension dans l'hydroxypropylcellulose à 0,2 % selon leur solubilité.

Leur activité sur les protéines de la phase aiguë a été évaluée par détermination des taux plasmatiques d'albumine 7 jours après l'induction de l'arthrite (méthode de dosage colorimétrique décrite par Lewis (Lewis EJ. & Coll., J. Pharmacol. Meth. 1989, 21, 183-94), l'adjuvant lui-même provoquant une chute de 31 % du taux d'albumine basal. L'atteinte clinique a été appréciée par mesure pléthysmométrique du volume des pattes postérieures et, à 14 jours, par cotation de l'ensemble des atteintes de la polyarthrite (rougeur et enflure de chaque articulation, nodules au niveau de la queue et des oreilles).

| Exemples | Correction de l'hypoalbuminémie |
|---|---|
| Exemple 1 | 64 % |
| Exemple 2 | 100 % |
| Exemple 3 | 25 % |
| Exemple 13 | 12 % |

L'activité du composé de l'exemple 2 sur ce paramètre est comparable à celle de la dexaméthasone (0,25 mg/kg/j.). Parallèlement, les produits tendent à diminuer l'intensité de l'oedème aigu au 6ème jour et s'opposent à la sévérité de la poylyarthrite observée 14 jours après l'injection d'adjuvant. C'est ainsi que le composé de l'exemple 2, comme la dexaméthasone, inhibe totalement (100 %) l'apparition de la polyarthrite à 14 jours. Le composé de l'exemple 26 inhibe, quant à lui, de 96 % l'apparition de celle-ci. Le romazarit, dans ces conditions, inhibe l'inhibition de la polyarthrite de 45 %.

### EXEMPLE 29 : Composition pharmaceutique

Formule de préparation pour 1000 comprimés dosés à 20 mg

| | |
|---|---|
| Composé de l'exemple 2 | 20 g |
| Hydroxypropylcellulose | 2 g |
| Amidon de blé | 10 g |
| Stéarate de magnésium | 100 g |
| Talc | 3 g |

## Revendications

1. Composés de formule générale (I) : 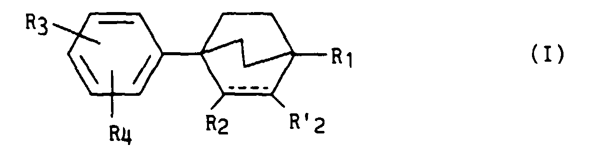 dans laquelle :
R₁ représente un atome d'halogène, un groupement hydroxy, alkyle (C₁-C₆) linéaire ou ramifié (éventuellement substitué par un ou plusieurs atomes d'halogène), alkoxy (C₁-C₆) linéaire ou ramifié (éventuellement substitué par un ou plusieurs atomes d'halogène), cyano, amino (substitué ou non par un ou deux groupements alkyle (C₁-C₆) linéaire ou ramifié), mercapto, alkylthio (C₁-C₆) linéaire ou ramifié ou phénoxy (substitué ou non par un ou plusieurs atomes ou groupements, identiques ou différents, d'halogène, hydroxy, alkyle (C₁-C₆) linéaire ou ramifié, alkoxy (C₁-C₆) linéaire ou ramifié ou trihalogénoalkyle (C₁-C₆) linéaire ou ramifié),
R₂ et R'₂ représentent deux atomes d'hydrogène dans le cas où le cycle bicyclooctane est insaturé,
ou bien
R₂ ou R'₂, dans le cas où le cycle bicyclooctane est saturé, identiques ou différents, représentent un atome d'hydrogène, d'halogène, un groupement hydroxy, alkyle (C₁-C₆) linéaire ou ramifié, alkoxy (C₁-C₆) linéaire ou ramifié, cyano, amino (substitué ou non par un ou deux groupements alkyle (C₁-C₆) linéaire ou ramifié) ou oxo,
R₃ représente l'un quelconque des groupements suivants :
. -CO₂H (à la condition que dans ce cas, R₁ soit différent d'un atome d'halogène, d'un groupement alkyle (C₁-C₆) linéaire, ou amino),
. CO - NH - CH₂ - CO₂H 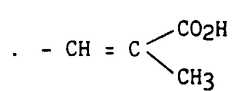
. - (CH₂)ₘ - CO₂H 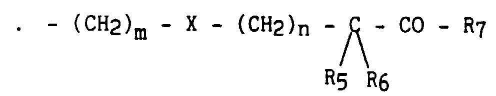 dans lesquels :
m représente 1, 2 ou 3,
X représente un atome d'oxygène, de soufre ou un groupement N-R (dans lequel R est un atome d'hydrogène ou un groupement alkyle (C₁-C₆) linéaire ou ramifié),
R₅ ou R₆, identiques ou différents, représentent un atome d'hydrogène, un groupement alkyle (C₁-C₆) linéaire ou ramifié, trifluorométhyle
ou 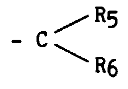 forme un radical cycloalkyle (C₃-C₆),
n représente 0, 1 ou 2,
R₇ représente un groupement hydroxy, alkoxy (C₁-C₆) linéaire ou ramifié, amino (substitué ou non par un ou deux groupements alkyle (C₁-C₆) linéaire ou ramifié), -O-CH₂-CO-NRR' (tels que R et R' représentent un groupement alkyle (C₁-C₆) linéaire ou ramifié, ou forment avec l'atome d'azote qui les portent un hétérocycle à 5 ou 6 chaînons),
. ou l'un quelconque des hétérocycles suivants : 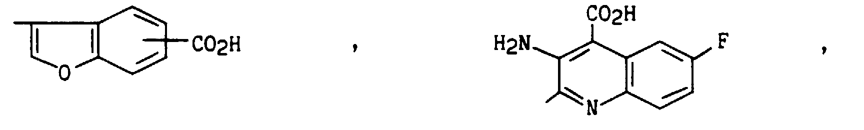 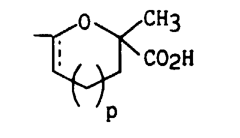 dans lequel p est égal à 0 ou 1, 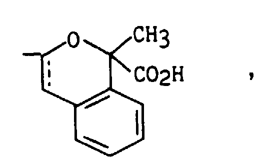
R₄ représente un atome d'hydrogène, d'halogène ou un groupement alkyle (C₁-C₆) linéaire ou ramifié, alkoxy (C₁-C₆) linéaire ou ramifié, trihalogénoalkyle (C₁-C₆),
les traits en pointillé sur les cycles indiquant la présence ou non d'une double liaison,
leurs énantiomères, diastéréoisomères et épimères ainsi que leurs sels d'addition à un acide ou à une base pharmaceutiquement acceptable.

2. Composés de formule (I) selon la revendication 1 dans laquelle le cycle bicyclooctane est saturé, leurs énantiomères, diastéréoisomères et épimères ainsi que leurs sels d'addition à un acide ou à une base pharmaceutiquement acceptable.

3. Composés de formule (I) selon la revendication 1 dans laquelle R₂ et R'₂ représentent deux atomes d'hydrogène, leurs énantiomères, diastéréoisomères et épimères ainsi que leurs sels d'addition à un acide ou à une base pharmaceutiquement acceptable.

4. Composés de formule (I) selon la revendication 1 qui est l'acide 2-méthyl-2-{[4-(4-méthoxybicyclo[2.2.2]oct-1-yl)phényl]méthoxy}propionique, ainsi que ses sels d'addition à une base pharmaceutiquement acceptable.

5. Procédé de préparation des composés de formule (I) selon la revendication 1 caractérisé en ce que l'on utilise comme produit de départ un composé de formule (II) : 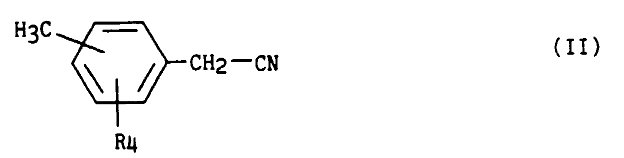 dans laquelle R₄ a la même signification que dans la formule (I),
que l'on fait réagir avec de l'acrylate de méthyle en présence d'une base pour donner le composé de formule (III) : 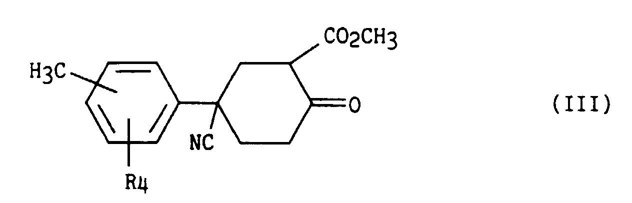 dans laquelle R₄ a la même signification que dans la formule (I),
que l'on soumet à l'action de l'acide paratoluènesulfonique dans l'éthylène glycol, pour conduire au composé de formule (IV) : 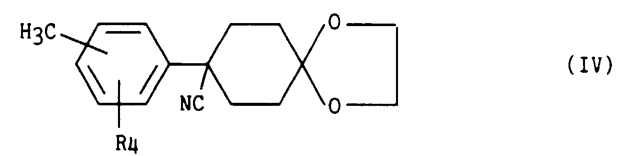 dans laquelle R₄ a la même signification que dans la formule (I),
qui subit l'action de l'iodure de méthyle magnésium, sous atmosphère inerte, pour conduire au composé de formule (V) : 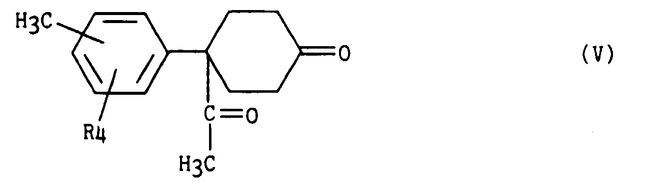 dans laquelle R₄ a la même signification que dans la formule (I),
que l'on fait réagir, en milieu anhydre, avec un alcool en milieu acide, pour conduire au composé de formule (VI) : 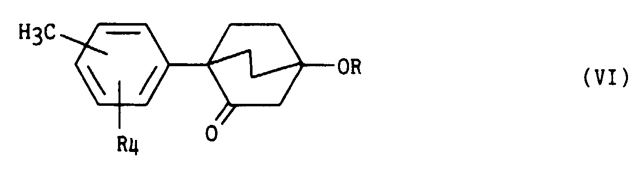 dans laquelle R₄ a la même signification que dans la formule (I) et R représente un groupement alkyle (C₁-C₆) linéaire ou ramifié,
composé de formule (VI) qui subit :
- soit une réduction totale en présence d'hydrate d'hydrazine et d'une base pour conduire au composé de formule (VIIa) : 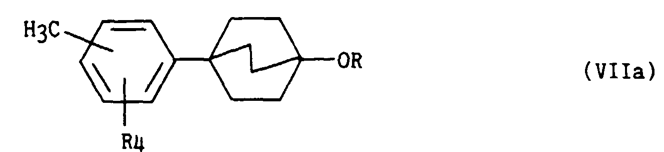 dans laquelle R₄ et R ont la même signification que précédemment,
- soit une réduction partielle en présence d'hydrure de lithium aluminium dans de l'éther suivie ou non d'une déshydratation pour conduire respectivement aux composés de formules (VIIb) et (VIIc) : 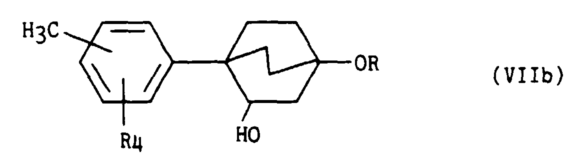 (composé de formule (VIIb) dont on transforme, si on le souhaite, le groupement hydroxy en atome d'halogène ou en groupement alkoxy, amino ou cyano, selon des techniques classiques de la chimie organique), 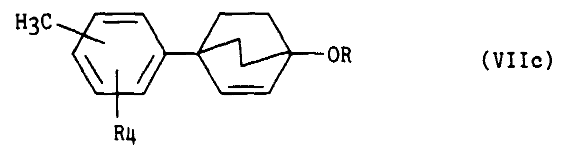 dans lesquelles R₄ et R ont la même signification que précédemment,
- soit l'action d'un ylure de phosphore suivie d'une réduction pour conduire au composé alkylé de formule (VIId) : 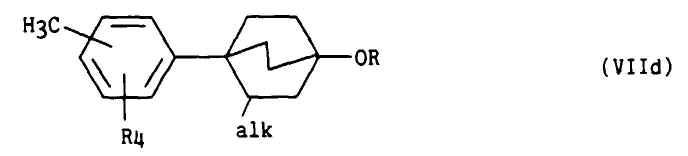 dans laquelle R₄ et R ont la même signification que précédemment et alk signifie alkyle (C₁-C₆) linéaire ou ramifié,
- soit, après transformation en énolate de lithium, une oxydation à l'aide de MoOPH (oxodiperoxymolybdenum-pyridine-hexaméthylphosphoramide) pour conduire au composé de formule (VIIe) : 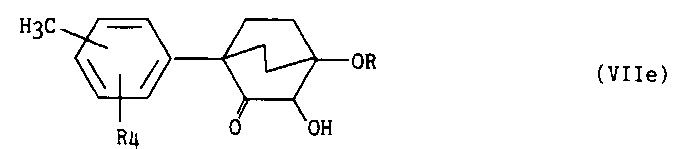 dans laquelle R₄ et R ont la même signification que précédemment (composé de formule (VIIe) dont on transforme, si on le souhaite, le groupement hydroxy en atome d'halogène ou en groupement alkoxy, amino ou cyano selon des techniques classiques de la chimie organique puis que l'on réduit éventuellement),
composés de formule (VIIa) à (VIIe) (dont on transforme, si on le souhaite, le groupement OR en groupement hydroxy ou en atome d'halogène puis amino, cyano, phénoxy, alkyle, mercapto, alkylthio, selon des techniques classiques de la chimie organique), qui constitue l'ensemble des composés de formule (VII) : 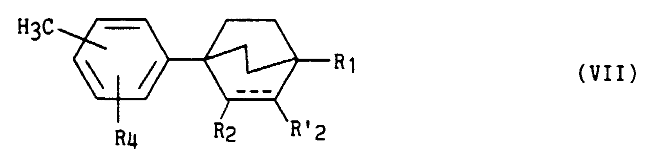 dans laquelle R₁, R₂, R'₂ et R₄ ont la même signification que dans la formule (I),
que l'on transforme en dérivé bromé correspondant de formule (VIIIa) par l'action du N-bromosuccinimide en milieu inerte : 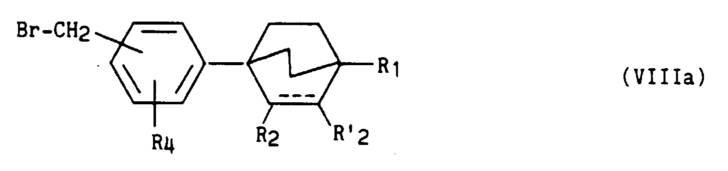 dans laquelle R₁, R₂, R'₂ et R₄ ont la même signification que dans la formule (I),
composé de formule (VIIIa), dont on transforme, éventuellement, le groupement -CH₂Br en groupement -CH₂CN correspondant puis en groupement -CH₂CO₂H, qui peut à nouveau subir des réactions classiques de la chimie organique conduisant :
- au composé de formule (I/a), cas particulier des composés de formule (I) : 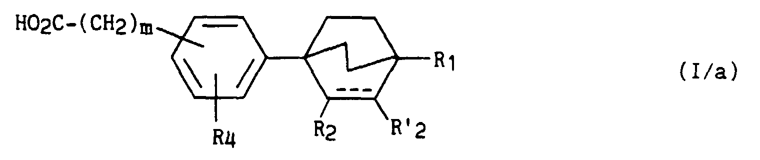 dans laquelle R₁, R₄, R₂, R'₂ et m ont la même signification que dans la formule (I),
- ou, au composé de formule (VIIIb) : 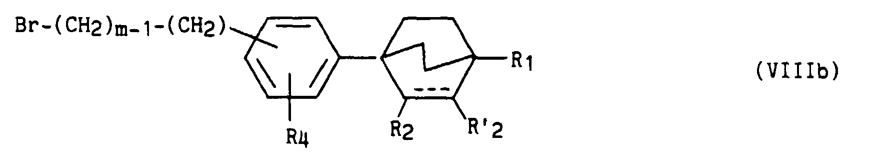 dans laquelle R₁, R₂, R'₂, R₄ et m ont la même signification que dans la formule (I),
composé de formule (VIIIa) ou (VIIIb) que l'on fait réagir :
a avec un composé de formule (IX) : 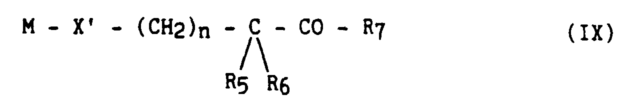 dans laquelle M représente un métal alcalin n, R₅, R₆ et R₇ sont tels que définis dans la formule (I) et X' représente un atome de soufre ou d'oxygène,
pour conduire au composé de formule (I/b), cas particulier des composés de formule (I) : 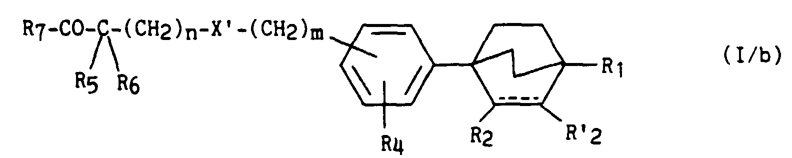 dans laquelle R₅, R₆, R₇, m, n, X', R₄, R₂, R'₂ et R₁ ont la même signification que précédemment,
dont on transforme, si on le souhaite, R₇ lorsqu'il représente un groupement hydroxy en groupement amino ou ester correspondant selon les techniques classiques de transformation de fonction acide en fonction amide,
b avec de l'hexaméthylènetétramine en milieu chroroformique, puis en milieu acétique,
pour conduire respectivement aux aldéhydes de formule (Xa) ou (Xb) : 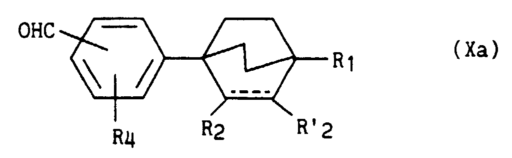 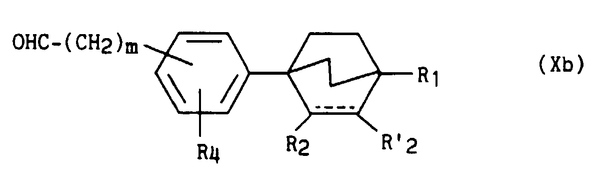
composé de formule (Xa) qui subit :
* soit l'action d'un oxydant comme le nitrate d'argent, pour conduire au composé de formule (I/c), cas particulier des composés de formule (I) : 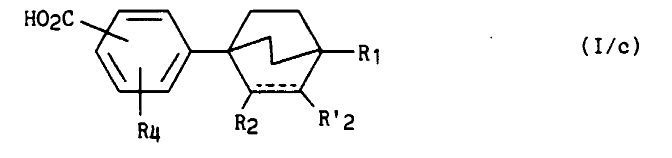 dans laquelle R₁, R₄, R₂ et R'₂ ont la même signification que précédemment,
que l'on peut faire éventuellement réagir :
- après transformation en chlorure d'acide, sur l'ylure de phosphore de formule (XI) : 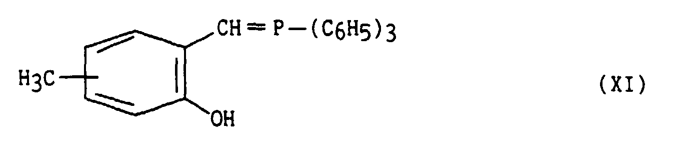 pour conduire au composé de formule (XII) : 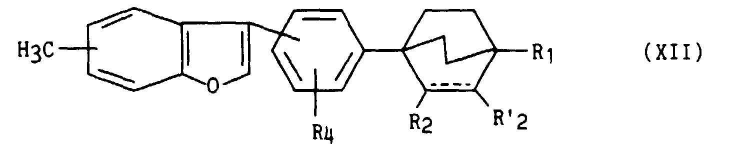 dans laquelle R₁, R₄, R₂ et R'₂ ont la même signification que précédemment,
que l'on transforme en composé de formule (I/d), cas particulier des composés de formule (I), selon les mêmes réactions que celles décrites pour le passage du composé de formule (VII) en composé de formule (I/c) via les composés de formule (VIIIa) et (Xa) : 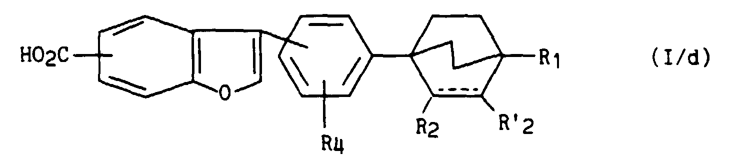 dans laquelle R₁, R₂, R'₂ et R₄ ont la même signification que précédemment,
- après transformation en chlorure d'acide, sur le diazométhane pour conduire à la diazométhyl cétone correspondante que l'on transforme en bromométhylcétone puis en aminométhylcétone pour conduire, après réaction avec la 5-fluoroisatine, au composé de formule (I/e), cas particulier des composés de formule (I) : 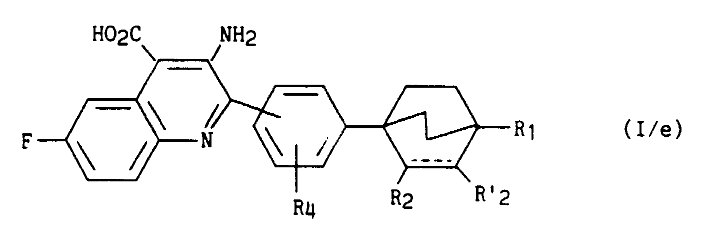 dans laquelle R₁, R₂, R'₂ et R₄ ont la même signification que dans la formule (I),
- ou, sur l'ester méthylique de la glycine selon la technique classique de couplage peptidique,
pour conduire au composé de formule (I/f), cas particulier des composés de formule (I) : 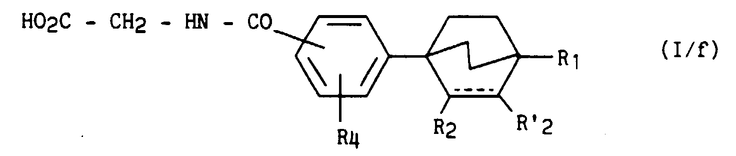
dans laquelle R₁, R₂, R'₂ et R₄ ont la même signification que dans la formule (I),
* soit l'action du magnésien C₆H₅CH₂MgBr suivie ou non d'une réaction d'oxydation puis de celle du pyruvate de méthyle en milieu acide, pour conduire après saponification au composé de formule (I/g), cas particulier des composés de formule (I) : 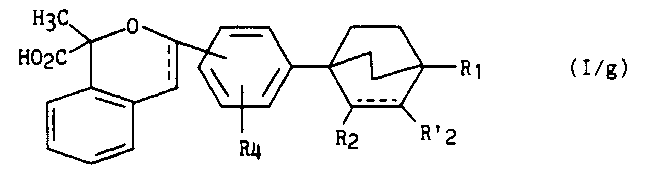 dans laquelle R₁, R₂, R'₂ et R₄ ont la même signification que dans la formule (I),
* soit l'action du magnésien CH₂=CH-MgBr suivie ou non d'une réaction d'oxydation puis de celle du pyruvate de méthyle en milieu acide pour conduire après saponification au composé de formule (I/h), cas particulier des composés de formule (I) : 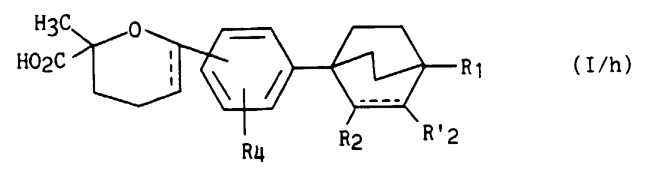 dans laquelle R₁, R₂, R'₂ et R₄ ont la même signification que dans la formule (I),
* soit l'action de : 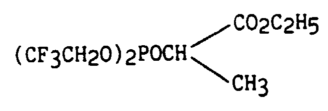 en présence d'une base,
pour conduire après saponification au composé de formule (I/i) : 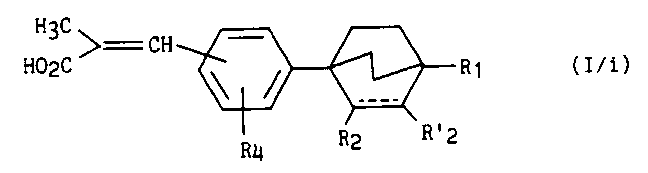 dans laquelle R₁, R₂, R'₂ et R₄ ont la même signification que dans la formule (I),
composés de formule (Xa) ou (Xb), qui peuvent subir l'action d'un amino ester de formule (XIII) : 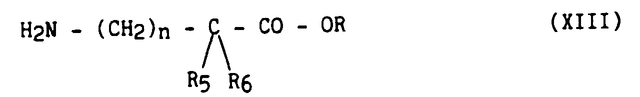 dans laquelle R représente un groupement alkyle, n, R₅ et R₆ ont la même signification que précédemment,
pour conduire au composé de formule (I/j), cas particulier des composés de formule (I) : 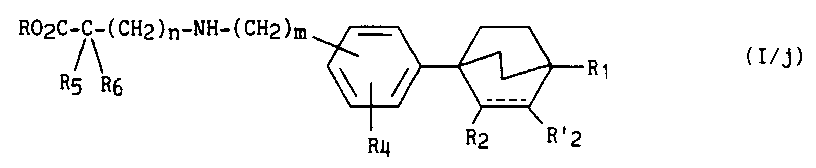 composé de formule (I/h) dont on transforme, si on le souhaite, la fonction acide ou fonction amide correspondante selon des techniques classiques de la chimie organique et la fonction amine secondaire en fonction amine tertiaire par alkylation,
composés de formule (I/a), (I/b), (I/c), (I/d), (I/e), (I/f), (I/g), (I/h), (I/i) ou (I/j) que l'on purifie, le cas échéant, selon une technique classique de purification dont on sépare, si on le souhaite, les isomères selon des techniques classiques de séparation, et que l'on transforme éventuellement en leurs sels d'addition à un acide ou à une base pharmaceutiquement acceptable.

6. Compositions pharmaceutiques contenant comme principe actif au moins un composé selon l'une quelconque des revendications 1 à 4 seul ou en combinaison avec un ou plusieurs véhicules inertes non toxiques pharmaceutiquement acceptables.

7. Composition pharmaceutique selon la revendication 6 contenant au moins un principe actif selon l'une quelconque des revendications 1 à 4 utiles dans le traitement de l'arthrite et des phénomènes inflammatoires dans lesquels les protéines de la phase aiguë se trouvent modifiées.

## Patentansprüche

1. Verbindungen der allgemeinen Formel (I): 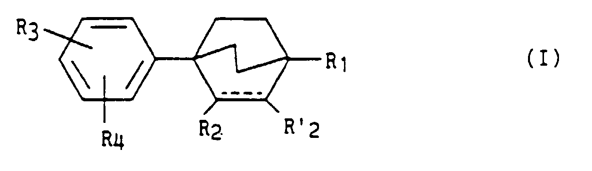 in der:
R₁ ein Halogenatom, eine Hydroxylgruppe, eine geradkettige oder verzweigte (gegebenenfalls durch eines oder mehrere Halogenatome substituierte) (C₁-C₆)-Alkylgruppe, eine geradkettige oder verzweigte (gegebenenfalls durch eines oder mehrere Halogenatome substituierte) (C₁-C₆)-Alkoxygruppe, eine Cyanogruppe, eine Aminogruppe (die gegebenenfalls durch ein oder zwei geradkettige oder verzweigte (C₁-C₆)-Alkylgruppen substituiert ist), eine Mercaptogruppe, eine geradkettige oder verzweigte (C₁-C₆)-Alkylthiogruppe oder eine Phenoxygruppe (die gegebenenfalls durch eine oder mehrere gleichartige oder verschiedene Halogenatome, Hydroxylgruppen, geradkettige oder verzweigte (C₁-C₆)-Alkylgruppen, geradkettige oder verzweigte (C₁-C₆)-Alkoxygruppen oder geradkettige oder verzweigte (C₁-C₆)-Trihalogenalkylgruppen substituiert ist),
R₂ und R'₂, wenn der Bicyclooctanring ungesättigt ist, zwei Wasserstoffatome oder
R₂ oder R'₂, wenn der Bicyclooctanring gesättigt ist, gleichartig oder verschieden sind und Wasserstoffatome, Halogenatome, Hydroxylgruppen, geradkettige oder verzweigte (C₁-C₆)-Alkylgruppen, geradkettige oder verzweigte (C₁-C₆)-Alkoxygruppen, Cyanogruppen, Aminogruppen (die gegebenenfalls durch eine oder zwei geradkettige oder verzweigte (C₁-C₆)-Alkylgruppen substituiert sind) oder Oxogruppen,
R₃ eine der folgenden Gruppen:
. - CO₂H (mit der Maßgabe, daß in diesem Fall R₁ von einem Halogenatom, einer geradkettigen (C₁-C₆)-Alkylgruppe oder einer Aminogruppe verschieden ist).
. -CO-NH-CH₂-CO₂H 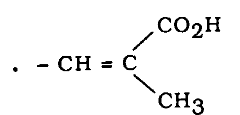
. - (CH₂)ₘ - C O₂H 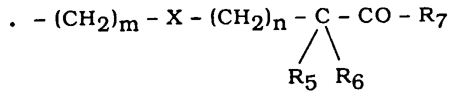 worin:
m 1, 2 oder 3,
X ein Sauerstoffatom, ein Schwefelatom oder eine Gruppe N-R (worin R ein Wasserstoffatom oder eine geradkettige oder verzweigte (C₁-C₆)-Alkylgruppe),
R₅ oder R₆, die gleichartig oder verschieden sein können, Wasserstoffatome, geradkettige oder verzweigte (C₁-C₆)-Alkylgruppen, Trifluormethylgruppen oder 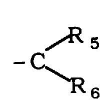 eine (C₃-C₆)-Cycloalkylgruppe bildet,
n 0, 1 oder 2 und
R₇ eine Hydroxylgruppe, eine geradkettige oder verzweigte (C₁-C₆) -Alkoxygruppe, eine Aminogruppe (die gegebenenfalls durch eine oder zwei geradkettige oder verzweigte (C₁-C₆)-Alkylgruppen substituiert ist), -O-CH₂-CO-NRR' (worin R und R' geradkettige oder verzweigte (C₁-C₆)-Alkylgruppen darstellen oder gemeinsam mit dem sie tragenden Stickstoffatom einen Heterocyclus mit 5 oder 6 Kettengliedern bilden) darstellen,
. oder einen der folgenden Heterocyclen: 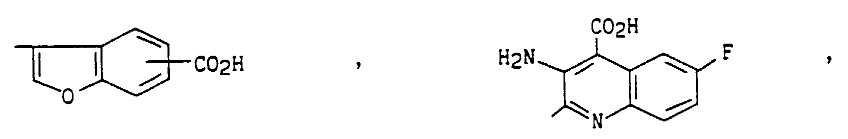 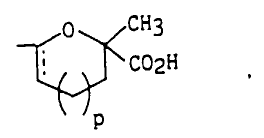 worin p 0 oder 1 darstellt, 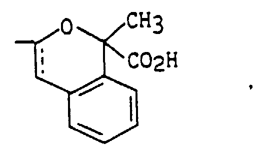 und
R₄ ein Wasserstoffatom, ein Halogenatom oder eine geradkettige oder verzweigte (C₁-C₆)-Alkylgruppe, eine geradkettige oder verzweigte (C₁-C₆)-Alkoxygruppe oder eine (C₁-C₆)-Trihalogenalkylgruppe bedeuten,
wobei die gepunkteten Linien in den Ringen angeben, daß gegebenenfalls eine Doppelbindung vorliegt,
deren Enantiomere, Diastereoisomere und Epimere sowie deren Additionssalze mit einer pharmazeutisch annehmbaren Säure oder Base.

2. Verbindungen der Formel (I) nach Anspruch 1, worin der Bicyclooctanring gesättigt ist, deren Enantiomere, Diastereoisomere und Epimere sowie deren Additionssalze mit einer pharmazeutisch annehmbaren Säure oder Base.

3. Verbindungen der Formel (I) nach Anspruch 1, worin R₂ und R'₂ zwei Wasserstoffatome bedeuten, deren Enantiomere, Diastereoisomere und Epimere sowie deren Additionssalze mit einer pharmazeutisch annehmbaren Säure oder Base.

4. Verbindungen der Formel (I) nach Anspruch 1, nämlich 2-Methyl-2-{(4-(4-methoxybicyclo[2.2.2]oct-1-yl)-phenyl]-methoxy}-propionsäure sowie deren Additionssalze mit einer pharmazeutisch annehmbaren Base.

5. Verfahren zur Herstellung der Verbindungen der Formel (I) nach Anspruch 1, **dadurch gekennzeichnet,** daß man als Ausgangsprodukt eine Verbindung der Formel (II): 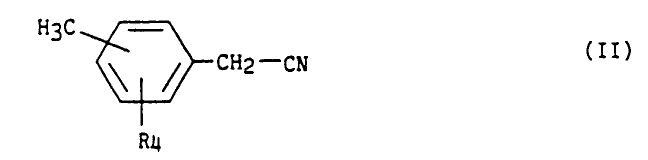 in der R₄ die bezüglich der Formel (I) angegebenen Bedeutungen besitzt, verwendet,
welches man in Gegenwart einer Base mit Acrylsäuremethylester umsetzt zur Bildung der Verbindung der Formel (III): 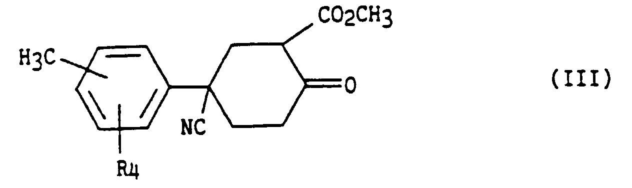 in der R₄ die bezüglich der Formel (I) angegebenen Bedeutungen besitzt,
welche man der Einwirkung von p-Toluolsulfonsäure in Ethylenglykol unterwirft zur Bildung der Verbindung der Formel (IV): 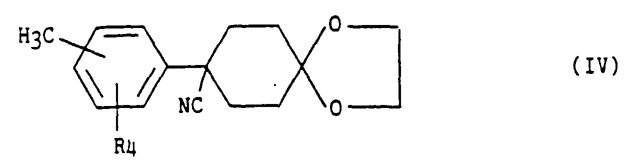 in der R₄ die bezüglich der Formel (I) angegebenen Bedeutungen besitzt,
welche man unter einer inerten Atmosphäre der Einwirkung von Methylmagnesiumiodid unterwirft zur Bildung der Verbindung der Formel (V): 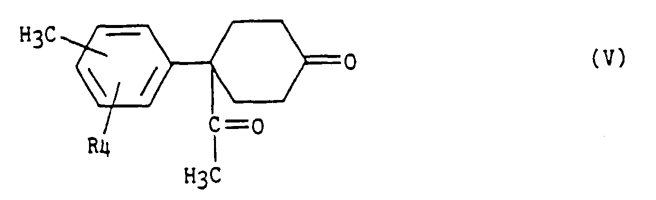 in der R₄ die bezüglich der Formel (I) angegebenen Bedeutungen besitzt,
welche man in wasserfreiem Medium mit einem Alkohol in saurem Medium umsetzt zur Bildung der Verbindung der Formel (VI): 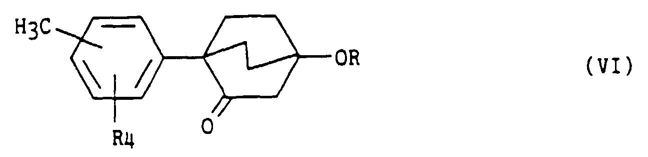 in der R₄ die bezüglich der Formel (I) angegebenen Bedeutungen besitzt und R eine geradkettige oder verzweigte (C₁-C₆)-Alkylgruppe darstellt,
welche Verbindung der Formel (VI) man:
- entweder einer vollständigen Reduktion in Gegenwart von Hydrazinhydrat und einer Base unterwirft zur Bildung der Verbindung der Formel (VIIa): 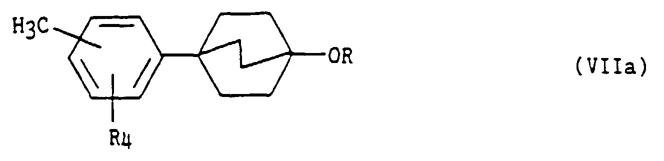 in der R₄ und R die oben angegebenen Bedeutungen besitzen,
- oder einer teilweisen Reduktion in Gegenwart von Lithiumaluminiumhydrid in Ether unterwirft, gegebenenfalls gefolgt von einer Dehydratation zur Bildung der Verbindungen der Formeln (VIIb) bzw. (VIIc): 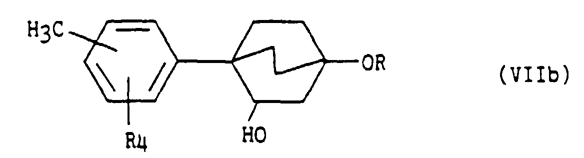 (wobei man gewünschtenfalls die Hydroxylgruppe der Verbindung der Formel (VIIb) unter Anwendung klassischer Methoden der organischen Chemie in ein Halogenatom oder eine Alkoxygruppe, Aminogruppe oder Cyanogruppe umwandelt), 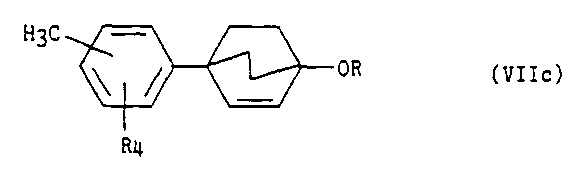 in der R₄ und R die oben angegebenen Bedeutungen besitzen,
- oder der Einwirkung eines Phosphorylids, gefolgt von einer Reduktion unterwirft zur Bildung der alkylierten Verbindung der Formel (VIId): 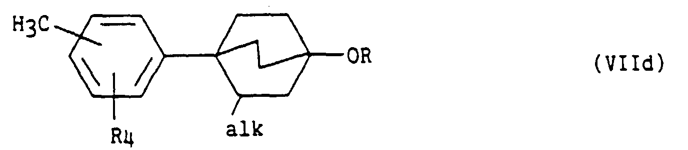 in der R₄ und R die oben angegebenen Bedeutungen besitzen und alk eine geradkettige oder verzweigte (C₁-C₆)-Alkylgruppe darstellt.
- oder nach der Umwandlung in das Lithiumenolat einer Oxidation mit Hilfe von MoOPH (Oxodiperoxymolybdän-pyridin-hexamethylphosphoramid) unterwirft zur Bildung der Verbindung der Formel (VIIe): 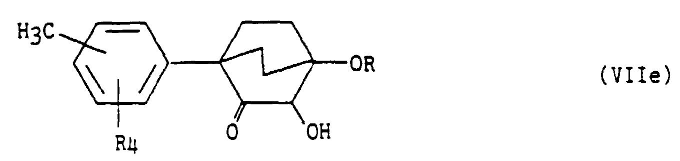 in der R₄ und R die oben angegebenen Bedeutungen besitzen (wobei man gewünschtenfalls die Hydroxylgruppe der Verbindung der Formel (VIIe) mit Hilfe klassischer Methoden der organischen Chemie in ein Halogenatom oder eine Alkoxygruppe, Aminogruppe oder Cyanogruppe umwandelt und welche man gegebenenfalls reduziert),
welche Verbindungen der Formeln (VIIa) bis (VIIe) (bei denen man gewünschtenfalls die OR-Gruppe mit Hilfe klassischer Methoden der organischen Chemie in eine Hydroxylgruppe oder ein Halogenatom und dann in eine Aminogruppe, Cyanogruppe, Phenoxygruppe, Alkylgruppe, Mercaptogruppe oder Alkylthiogruppe umwandelt), die Gesamtheit der Verbindungen der Formel (VII) darstellen: 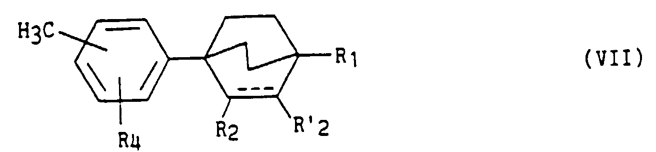 in der R₁, R₂, R'₂ und R₄ die bezüglich der Formel (I) angegebenen Bedeutungen besitzen,
welche man durch Einwirkung von N-Bromsuccinimid in inertem Medium in das entsprechende Bromderivat der Formel (VIIIa) umwandelt: 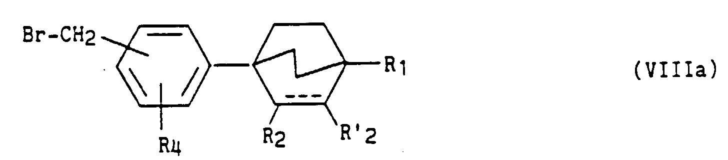 in der R₁, R₂, R'₂ und R₄ die bezüglich der Formel (I) angegebenen Bedeutungen besitzen,
wobei man gegebenenfalls die Gruppe -CH₂Br der Verbindung der Formel (VIIIa) in die entsprechende Gruppe -CH₂CN umwandelt und dann in die Gruppe -CH₂CO₂H, welche erneut klassischen Reaktionen der organischen Chemie unterworfen werden kann zur Bildung:
- der Verbindung der Formel (I/a), einem Sonderfall der Verbindungen der Formel (I) : 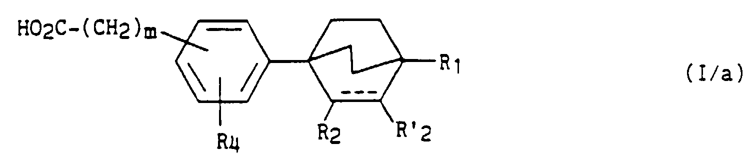 in der R₁, R₄, R₂, R'₂ und m die bezüglich der Formel (I) angegebenen Bedeutungen besitzen,
- oder der Verbindung der Formel (VIIIb): 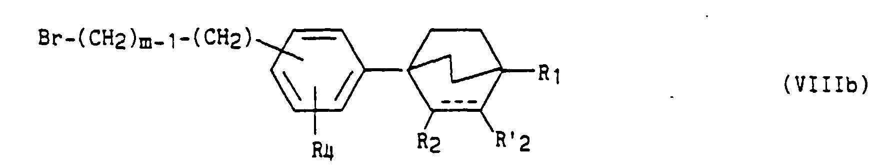 in der R₁, R₂, R'₂, R₄ und m die bezüglich der Formel (I) angegebenen Bedeutungen besitzen,
welche Verbindung der Formel (VIIIa) oder (VIIIb) man:
a mit einer Verbindung der Formel (IX): 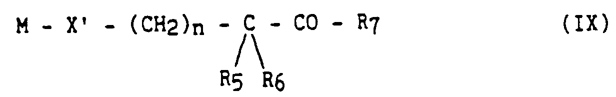 in der M ein Alkalimetall darstellt, n, R₅, R₆ und R₇ die bezüglich der Formel (I) angegebenen Bedeutungen besitzen und X' ein Schwefelatom oder ein Sauerstoffatom darstellt,
umsetzt zur Bildung der Verbindung der Formel (I/b), einem Sonderfall der Verbindungen der Formel (I): 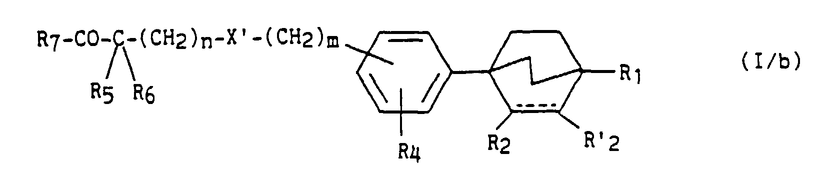 in der R₅, R₆, R₇, m, n, X', R₄, R₂, R'₂ und R₁ die oben angegebenen Bedeutungen besitzen,
bei welchen man gewünschtenfalls R₇, wenn es eine Hydroxylgruppe darstellt, unter Anwendung klassischer Methoden der Umwandlung einer Säurefunktion in die Amidfunktion in die entsprechende Aminogruppe oder Estergruppe umwandelt,
b mit Hexamethylentetramin in Chloroformmedium und dann in essigsaurem Medium umsetzt,
zur Bildung der Aldehyde der Formel (Xa) bzw. (Xb): 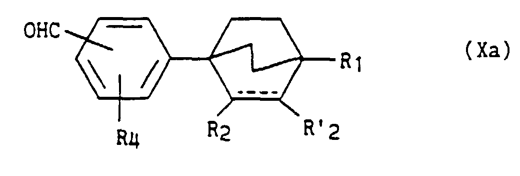 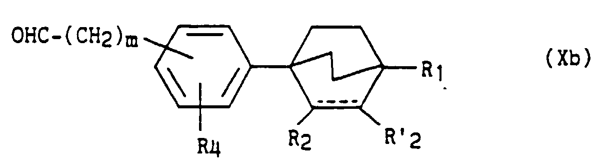
welche Verbindung der Formel (Xa) man:
* entweder der Einwirkung eines Oxidationsmittels, wie Silbernitrat, unterwirft zur Bildung der Verbindung der Formel (I/c), einem Sonderfall der Verbindungen der Formel (I): 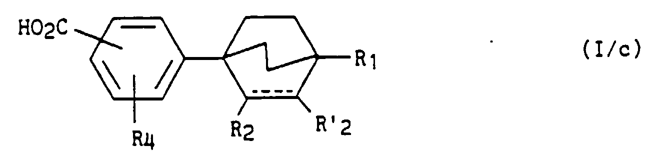 in der R₁, R₄, R₂ und R'₂ die oben angegebenen Bedeutungen besitzen, welche man gegebenenfalls:
- nach der Umwandlung in das Säurechlorid mit dem Phosphorylid der Formel (XI) umsetzt: 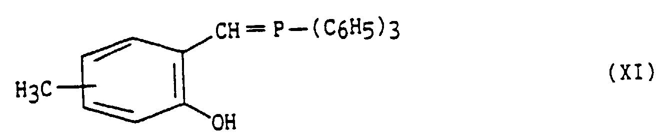 zur Bildung der Verbindung der Formel (XII): 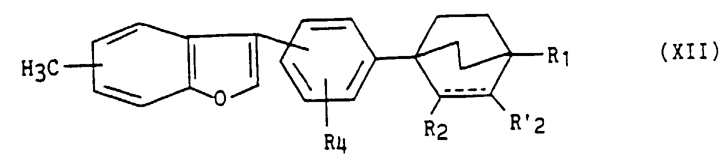 in der R₁, R₄, R₂ und R'₂ die oben angegebenen Bedeutungen besitzen,
welche man in die Verbindung der Formel (I/d) umwandelt, einem Sonderfall der Verbindungen der Formel (I), unter Anwendung der gleichen Reaktionen, wie sie für die Umwandlung der Verbindung der Formel (VII) in die Verbindung der Formel (I/c) über die Verbindungen der Formeln (VIIIa) und (Xa) beschrieben sind: 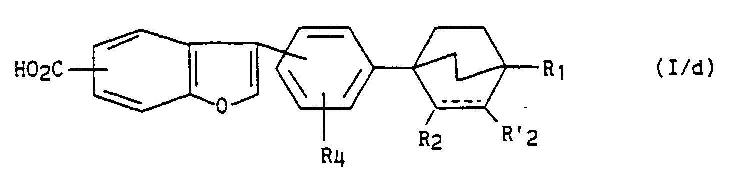 in der R₁, R₂, R'₂ und R₄ die oben angegebenen Bedeutungen besitzen,
- nach der Umwandlung in das Säurechlorid mit Diazomethan umsetzt zur Bildung des entsprechenden Diazomethylketons, welches man in das Brommethylketon und dann in das Aminomethylketon umwandelt, so daß man nach der Reaktion mit 5-Fluorisatin die Verbindung der Formel (I/e) erhält, einem Sonderfall der Verbindungen der Formel (I): 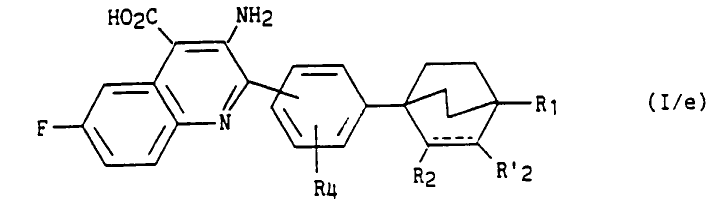 in der R₁, R₂, R'₂ und R₄ die bezüglich der Formel (I) angegebenen Bedeutungen besitzen,
- oder mit dem Glycinmethylester unter Anwendung einer klassischen Peptidkupplungstechnik umsetzt zur Bildung der Verbindung der Formel (I/f), einem Sonderfall der Verbindungen der Formel (I): 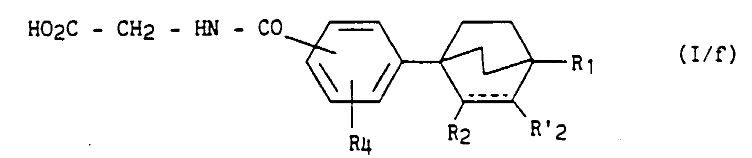 in der R₁, R₂, R'₂ und R₄ die bezüglich der Formel (I) angegebenen Bedeutungen besitzen,
* oder der Einwirkung der Magnesiumverbindung C₆H₅CH₂MgBr unterwirft, gegebenenfalls gefolgt von einer Oxidationsreaktion und dann einer Reaktion mit Methylpyruvat in saurem Medium, so daß man nach dem Verseifen die Verbindung der Formel (I/g) erhält, einem Sonderfall der Verbindungen der Formel (I): 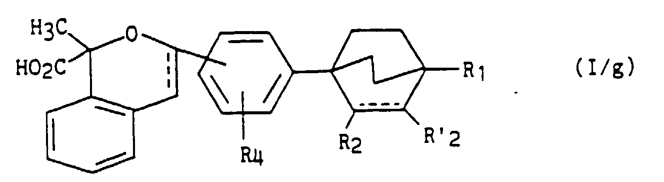 in der R₁, R₂, R'₂ und R₄ die bezüglich der Formel (I) angegebenen Bedeutungen besitzen,
* oder der Einwirkung der Magnesiumverbindung CH₂=CH-MgBr unterwirft, gegebenenfalls gefolgt von einer Oxidationsreaktion und dann einer Reaktion mit Methylpyruvat in Gegenwart eines sauren Mediums, so daß man nach der Verseifung die Verbindung der Formel (I/h) erhält, einem Sonderfall der Verbindungen der Formel (I): 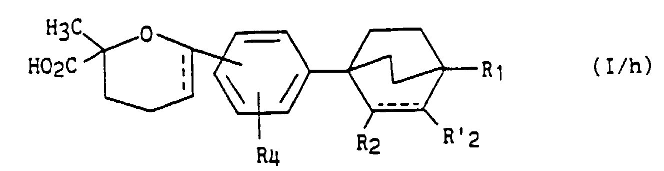 in der R₁, R₂, R'₂ und R₄ die bezüglich der Formel (I) angegebenen Bedeutungen besitzen,
* oder der Einwirkung von: 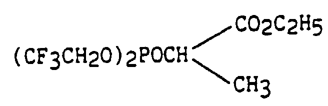 in Gegenwart einer Base unterwirft, so daß man nach der Verseifung die Verbindung der Formel (I/i) erhält: 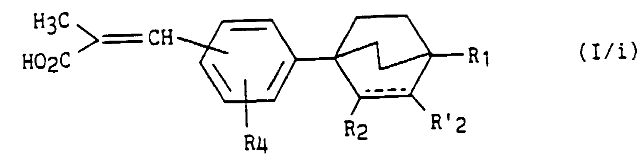 in der R₁, R₂, R'₂ und R₄ die bezüglich der Formel (I) angegebenen Bedeutungen besitzen,
welche Verbindungen der Formeln (Xa) oder (Xb) der Einwirkung eines Aminoesters der Formel (XIII): 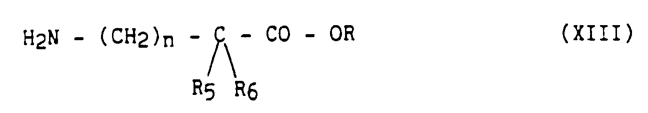 in der R eine Alkylgruppe darstellt und n, R₅ und R₆ die oben angegebenen Bedeutungen besitzen, unterzogen werden können,
so daß man die Verbindung der Formel (I/j) erhält, einem Sonderfall der Verbindungen der Formel (I): 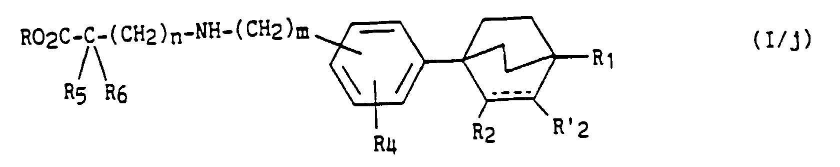 wobei man gewünschtenfalls die Säurefunktion der Verbindung der Formel (I/h) unter Anwendung klassischer Methoden der organischen Chemie in die entsprechende Amidfunktion umwandelt und die sekundäre Aminofunktion durch Alkylierung in eine tertiäre Aminofunktion,
welche Verbindungen der Formeln (I/a), (I/b), (I/c), (I/d), (I/e), (I/f), (I/g), (I/h), (I/i) und (I/j) man gegebenenfalls mit Hilfe klassischer Reinigungsmethoden reinigt und gewünschtenfalls deren Isomeren mit Hilfe klassischer Trennungsmethoden trennt und gegebenenfalls mit Hilfe einer pharmazeutisch annehmbaren Säure oder Base in ihre Additionssalze umwandelt.

6. Pharmazeutische Zubereitungen enthaltend als Wirkstoff mindestens eine Verbindung nach einem der Ansprüche 1 bis 4 allein oder in Kombination mit einem oder mehreren inerten, nichttoxischen, pharmazeutisch annehmbaren Trägermaterialien.

7. Pharmazeutische Zubereitung nach Anspruch 6 enthaltend mindestens einen Wirkstoff nach einem der Ansprüche 1 bis 4 zur Behandlung der Arthritis und von Entzündungsphänomenen, bei denen die Proteine der akuten Phase modifiziert werden.

## Claims

1. Compounds of the general formula (I) : 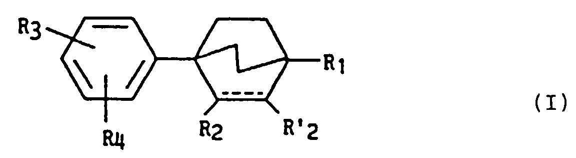 wherein :
R₁ represents a halogen atom, a hydroxy group, a straight-chain or branched (C₁-C₆)alkyl group (optionally substituted by one or more halogen atoms), a straight-chain or branched (C₁-C₆)alkoxy group (optionally substituted by one or more halogen atoms), a cyano group, an amino group (unsubstituted or substituted by one or two straight-chain or branched (C₁-C₆)alkyl groups), a mercapto group, a straight-chain or branched (C₁-C₆)alkylthio group or a phenoxy group (unsubstituted or substituted by one or more atoms or groups that are identical or different selected from halogen, hydroxy, straight-chain (C₁-C₆)alkyl, branched (C₁-C₆)alkyl, straight-chain (C₁-C₆)alkoxy, branched (C₁-C₆)-alkoxy, straight-chain trihalo-(C₁-C₆)alkyl and branched trihalo-(C₁-C₆)alkyl),
R₂ and R'₂ represent two hydrogen atoms when the bicyclooctane ring is unsaturated,
or
R₂ or R'₂, when the bicyclooctane ring is saturated, are identical or different and each represents a hydrogen atom, a halogen atom, a hydroxy group, a straight-chain or branched (C₁-C₆)alkyl group , a straight-chain or branched (C₁-C₆)alkoxy group, a cyano group, an amino group (unsubstituted or substituted by one or two straight-chain or branched (C₁-C₆)alkyl groups), or an oxo group,
R₃ represents any one of the following groups :
. -CO₂H (provided that in that case R₁ is other than a halogen atom, a straight-chain (C₁-C₆)alkyl group or amino),
. -CO-NH-CH₂-CO₂H 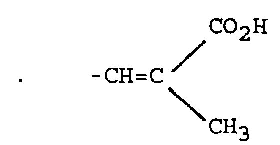
. - (CH₂)ₘ-CO₂H 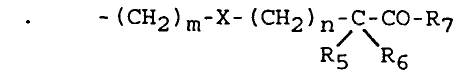 wherein :
m represents 1, 2 or 3,
X represents an oxygen atom, a sulphur atom or an N-R group (wherein R is a hydrogen atom or a straight-chain or branched (C₁-C₆)alkyl group),
R₅ or R₆, which are identical or different, each represents a hydrogen atom, a straight-chain or branched (C₁-C₆)alkyl group, or a trifluoromethyl group or 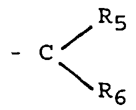 forms a (C₃-C₆)cycloalkyl radical,
n represents 0, 1 or 2,
R₇ represents a hydroxy group, a straight-chain or branched (C₁-C₆)alkoxy group, an amino group (unsubstituted or substituted by one or two straight-chain or branched (C₁-C₆)alkyl groups), -O-CH₂-CO-NRR' (such that R and R' represent a straight-chain or branched (C₁-C₆)alkyl group, or form with the nitrogen atom carrying them a heterocycle having 5 or 6 chain members),
. or any one of the following heterocycles : 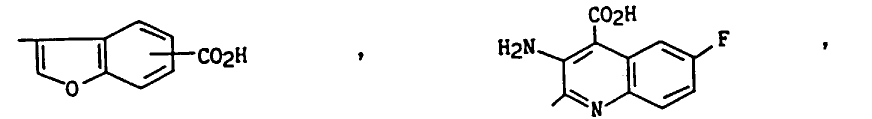 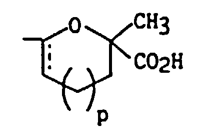 wherein p is 0 or 1, 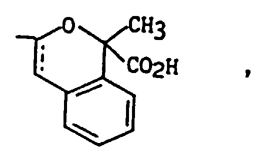
R₄ represents a hydrogen atom, a halogen atom or a straight-chain or branched (C₁-C₆)alkyl group, a straight-chain or branched (C₁-C₆)alkoxy group or a trihalo-(C₁-C₆)alkyl group,
the broken lines in the rings indicating the presence or absence of a double bond,
their enantiomers, diastereoisomers and epimers, and also the addition salts thereof with a pharmaceutically acceptable acid or base.

2. Compounds of formula (I) according to claim 1 wherein the bicyclooctane ring is saturated, their enantiomers, diastereoisomers and epimers, and also the addition salts thereof with a pharmaceutically acceptable acid or base.

3. Compounds of formula (I) according to claim 1 wherein R₂ and R'₂ represent two hydrogen atoms, their enantiomers, diastereoisomers and epimers, and also the addition salts thereof with a pharmaceutically acceptable acid or base.

4. Compound of formula (I) according to claim 1 which is 2-methyl-2-{[4-(4-methoxybicyclo[2.2.2]oct-1-yl)phenyl]-methoxy}propionic acid, and the addition salts thereof with a pharmaceutically acceptable base.

5. Process for the preparation of compounds of formula (I) according to claim 1, characterised in that there is used as starting material a compound of formula (II) : 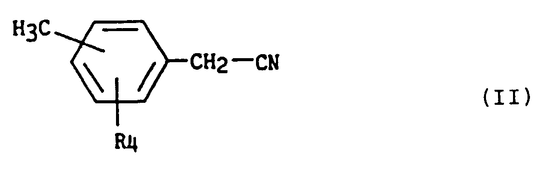 wherein R₄ is as defined for formula (I),
which is reacted with methyl acrylate in the presence of a base to yield a compound of formula (III) : 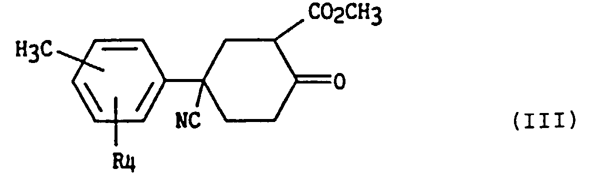 wherein R₄ is as defined for formula (I),
which is subjected to the action of para-toluenesulphonic acid in ethylene glycol to yield a compound of formula (IV) : 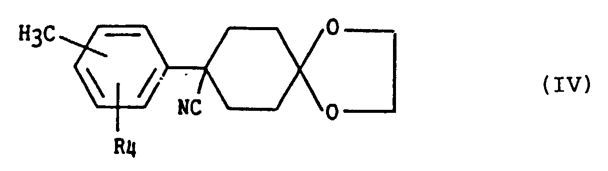 wherein R₄ is as defined for formula (I),
which is subjected to the action of methylmagnesium iodide, under an inert atmosphere, to yield a compound of formula (V) : 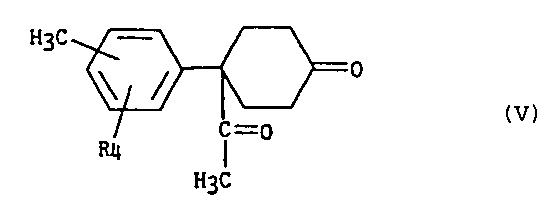 wherein R₄ is as defined for formula (I),
which is reacted, in an anhydrous medium, with an alcohol in acid medium, to yield a compound of formula (VI) : 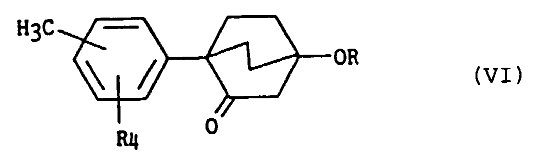 wherein R₄ is as defined for formula (I) and R represents a straight-chain or branched (C₁-C₆)alkyl group,
which compound of formula (VI) is subjected :
- to total reduction in the presence of hydrazine hydrate and a base to yield a compound of formula (VIIa) : 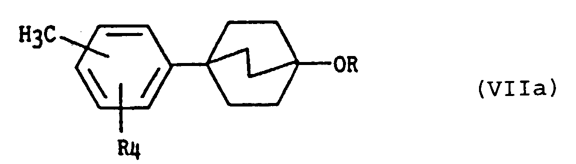 wherein R₄ and R are as defined hereinbefore,
- or to partial reduction in the presence of lithium aluminium hydride in ether which may or may not be followed by dehydration to yield, as appropriate, compounds of formula (VIIb) and (VIIc) : 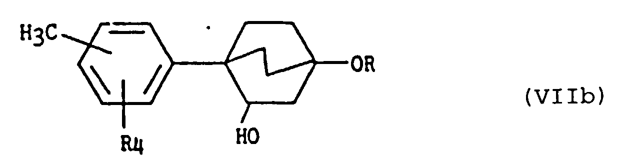 (the hydroxy group of which compound of formula (VIIb) is converted, if desired, into a halogen atom or into an alkoxy, amino or cyano group according to conventional methods of organic chemistry), 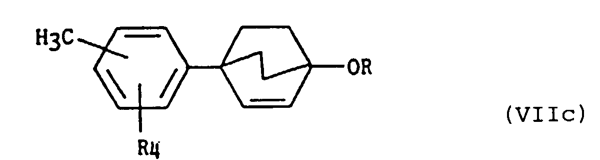 wherein R₄ and R are as defined hereinbefore,
- or to the action of a phosphorus ylid, followed by reduction, to yield an alkylated compound of formula (VIId) : 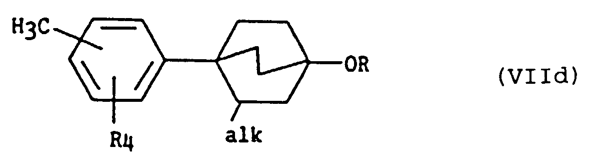 wherein R₄ and R are as defined hereinbefore and alk denotes straight-chain or branched (C₁-C₆)alkyl,
- or, after conversion into the lithium enolate, to oxidation with the aid of MoOPH (oxodiperoxymolybdenum(pyridine)hexamethylphosphoramide) to yield the compound of formula (VIIe) : 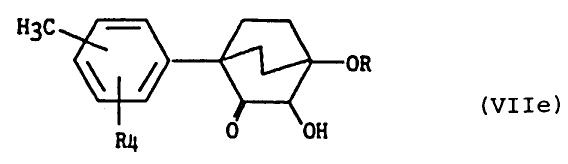 wherein R₄ and R are as defined hereinbefore, (the hydroxy group of which compound of formula (VIIe) is converted, if desired, into a halogen atom or into an alkoxy, amino or cyano group according to conventional methods of organic chemistry, and then optionally reduced),
which compounds of formulae (VIIa) to (VIIe) (the OR group of which, if desired, is converted into a hydroxy group or into a halogen atom then amino, cyano, phenoxy, alkyl, mercapto or alkylthio, according to conventional methods of organic chemistry), constitute the totality of the compounds of formula (VII) : 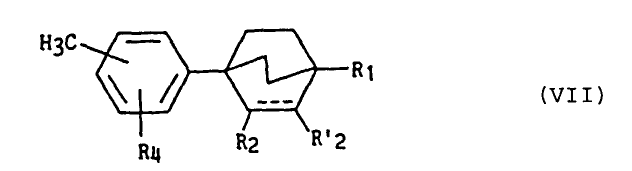 wherein R₁, R₂, R'₂ and R₄ are as defined for formula (I),
which compounds are converted into the corresponding brominated compounds of formula (VIIIa) : 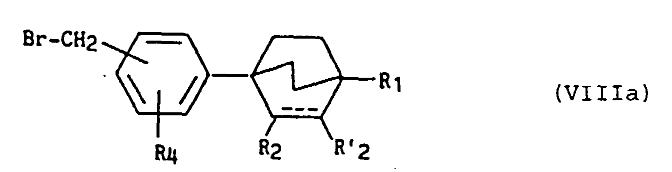 wherein R₁, R₂, R'₂ and R₄ are as defined for formula (I), by the action of N-bromosuccinimide in an inert medium,
the -CH₂Br group of which compound of formula (VIIIa) is converted, if desired, into the corresponding -CH₂CN group and then into the -CH₂CO₂H group, which may again be subjected to conventional reactions of organic chemistry yielding :
- a compound of formula (I/a) : 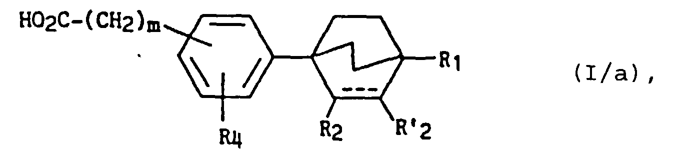 a particular case of compounds of formula (I), wherein R₁, R₄, R₂, R'₂ and m are as defined for formula (I),
- or a compound of formula (VIIIb) : 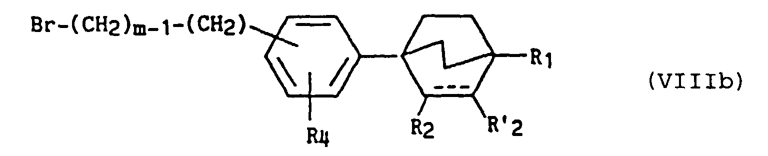 wherein R₁, R₂, R'₂, R₄ and m are as defined for formula (I),
which compound of formula (VIIIa) or (VIIIb) is reacted :
a with a compound of formula (IX) : 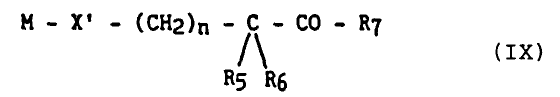 wherein M represents an alkali metal, n, R₅, R₆ and R₇ are as defined for formula (I) and X' represents a sulphur or oxygen atom, to yield a compound of formula (I/b) : 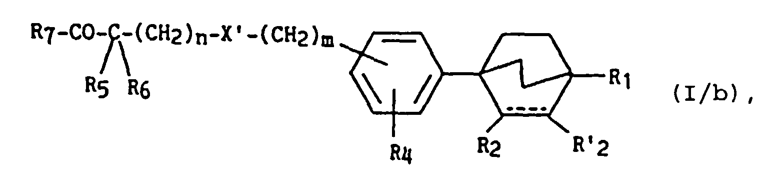 a particular case of compounds of formula (I), wherein R₅, R₆, R₇, m, n, X', R₄, R₂, R'₂ and R₁ are as defined hereinbefore,
the group R₇ of which, when it represents a hydroxy group, is converted, if desired, into an amino or corresponding ester group according to conventional methods of conversion of the acid function into the amide function,
b with hexamethylenetetramine in a chloroform medium, then an acetic medium,
to yield, respectively, the aldehydes of formula (Xa) or (Xb) : 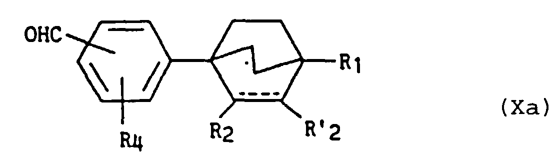 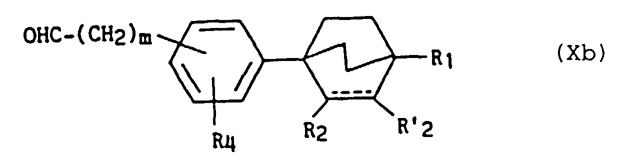
which compound of formula (Xa) is subjected :
* to the action of an oxidising agent such as silver nitrate,
to yield the compound of formula (I/c) : 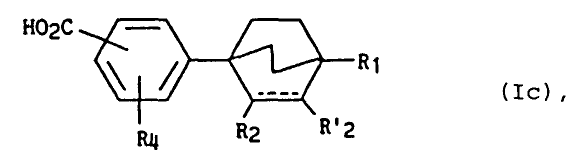 a particular case of compounds of formula (I), wherein R₁, R₄, R₂ and R'₂ are as defined hereinbefore,
which may optionally be reacted :
- after conversion into the acid chloride, with the phosphorus ylid of formula (XI) 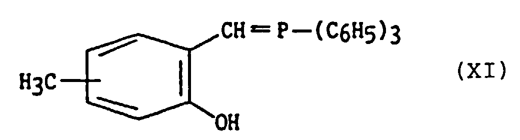 to yield a compound of formula (XII) 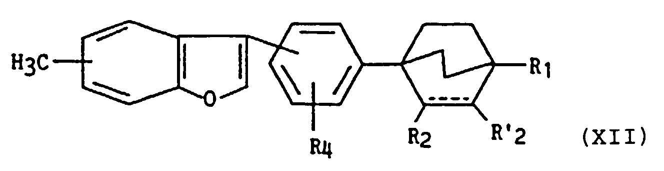 wherein R₁, R₄, R₂ and R'₂ are as defined hereinbefore,
which is converted into a compound of formula (I/d) 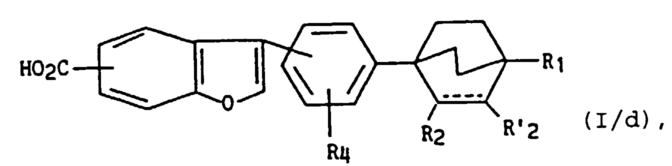 a particular case of compounds of formula (I), wherein R₁, R₂, R'₂ and R₄ are as defined hereinbefore, in accordance with the same reactions as those described for the conversion of the compound of formula (VII) into a compound of formula (I/c) via the compounds of formula (VIIIa) and (Xa),
- after conversion into the acid chloride, with diazomethane to yield the corresponding diazomethyl ketone, which is converted into the bromomethyl ketone and then into the aminomethyl ketone to yield, after reaction with 5-fluoroisatin, a compound of formula (I/e) : 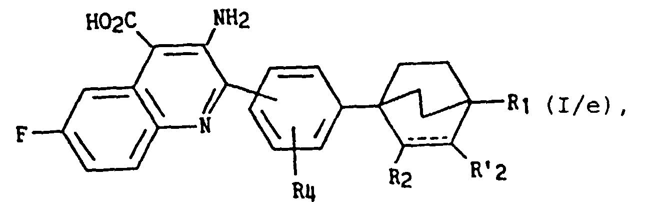 a particular case of compounds of formula (I), wherein R₁, R₂, R'₂ and R₄ are as defined for formula (I),
- or with glycine methyl ester according to the conventional peptide coupling technique, to yield a compound of formula (I/f) 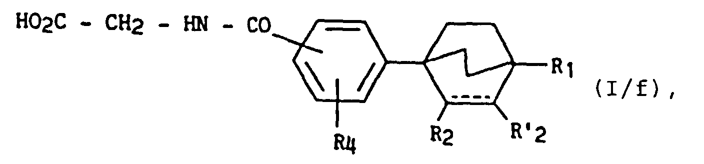 a particular case of compounds of formula (I), wherein R₁, R₂, R'₂ and R₄ are as defined for formula (I),
* or to the action of the magnesium compound C₆H₅CH₂MgBr, which may or may not be followed by oxidation, and then to the action of methyl pyruvate in acid medium to yield, after hydrolysis, a compound of formula (I/g) : 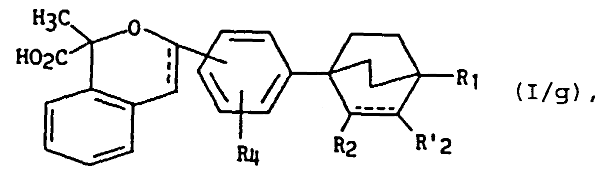 a particular case of compounds of formula (I), wherein R₁, R₂, R'₂ and R₄ are as defined for formula (I),
* or to the action of the magnesium compound CH₂=CH-MgBr, which may or may not be followed by oxidation, and then to the action of methyl pyruvate in acid medium to yield, after hydrolysis, a compound of formula (I/h) : 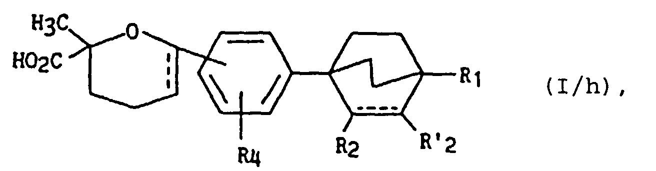 a particular case of compounds of formula (I), wherein R₁, R₂, R'₂ and R₄ are as defined for formula (I),
* or to the action of : 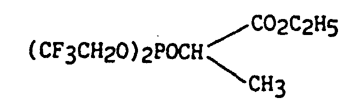 in the presence of a base to yield, after hydrolysis, a compound of formula (I/i) : 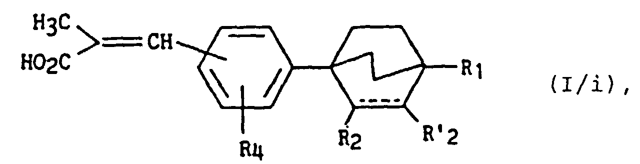 wherein R₁, R₂, R'₂ and R₄ are as defined for formula (I), or
which compounds of formula (Xa) or (Xb) may be subjected to the action of an amino ester of formula (XIII) : 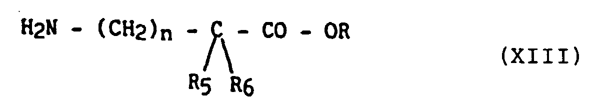 wherein R represents an alkyl group and n, R₅ and R₆ are as defined hereinbefore,
to yield a compound of formula (I/j) : 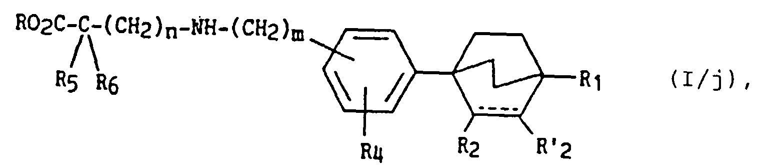 a particular case of compounds of formula (I), the acid function or corresponding amide function of which compound of formula (I/j) is converted, if desired, according to conventional methods of organic chemistry, and the secondary amine function of which is converted, if desired, into the tertiary amine function by alkylation,
which compounds of formulae (I/a), (I/b), (I/c), (I/d), are purified, where appropriate, according to a conventional purification technique and, if desired, are separated into their isomers according to conventional separation techniques, and are optionally converted into their addition salts with a pharmaceutically acceptable acid or base.

6. Pharmaceutical compositions comprising as active ingredient at least one compound according to any one of claims 1 to 4 alone or in combination with one or more pharmaceutically acceptable, inert, non-toxic carriers.

7. Pharmaceutical composition according to claim 6 comprising at least one active ingredient according to any one of claims 1 to 4 for use in the treatment of arthritis and inflammatory conditions in which the proteins of the acute phase are found to be modified.
